(19) Europäisches Patentamt
European Patent Office
Office européen des brevets

(11) Publication number : **0 465 426 A1**

# (12) EUROPEAN PATENT APPLICATION

(21) Application number : **91810507.3**

(22) Date of filing : **28.06.91**

(51) Int. Cl.$^5$ : **C07H 19/01**, A61K 31/70

(30) Priority : **02.07.90 DE 4021191**
**30.10.90 DE 4034480**
**30.10.90 DE 4034479**
**30.10.90 DE 4034478**
**30.10.90 DE 4034477**
**30.10.90 DE 4034476**
**08.05.91 DE 4115132**

(43) Date of publication of application :
**08.01.92 Bulletin 92/02**

(84) Designated Contracting States :
**AT BE CH DE DK ES FR GB GR IT LI LU NL SE**

(71) Applicant : **SANDOZ LTD.**
**Lichtstrasse 35**
**CH-4002 Basel (CH)**
(84) **BE CH DK ES FR GB GR IT LI LU NL SE**

(71) Applicant : **SANDOZ-PATENT-GMBH**
**Humboldtstrasse 3**
**W-7850 Lörrach (DE)**
(84) **DE**

(71) Applicant : **SANDOZ-ERFINDUNGEN**
**Verwaltungsgesellschaft m.b.H.**
**Brunner Strasse 59**
**A-1235 Wien (AT)**
(84) **AT**

(72) Inventor : **Baumann, Karl**
**Simmeringer Hauptstrasse 36/2/29**
**A-1110 Vienna (AT)**

## (54) Heteroatoms-containing tricyclic compounds.

(57) The invention relates to the field of macrolides. It concerns 12-(2'-cyclopentylvinyl)-11,28-dioxa-4-azatricyclo[22.3.1.0$^{4,9}$]octacos-18-ene or -14,18-diene and 11-(2'-cyclopentylvinyl)-10,27-dioxa-4-azatricyclo[21.3.1.0$^{4,8}$]heptacos-17-ene or -13,17-diene derivatives, in particular the compounds of formula I

EP 0 465 426 A1

Jouve, 18, rue Saint-Denis, 75001 PARIS

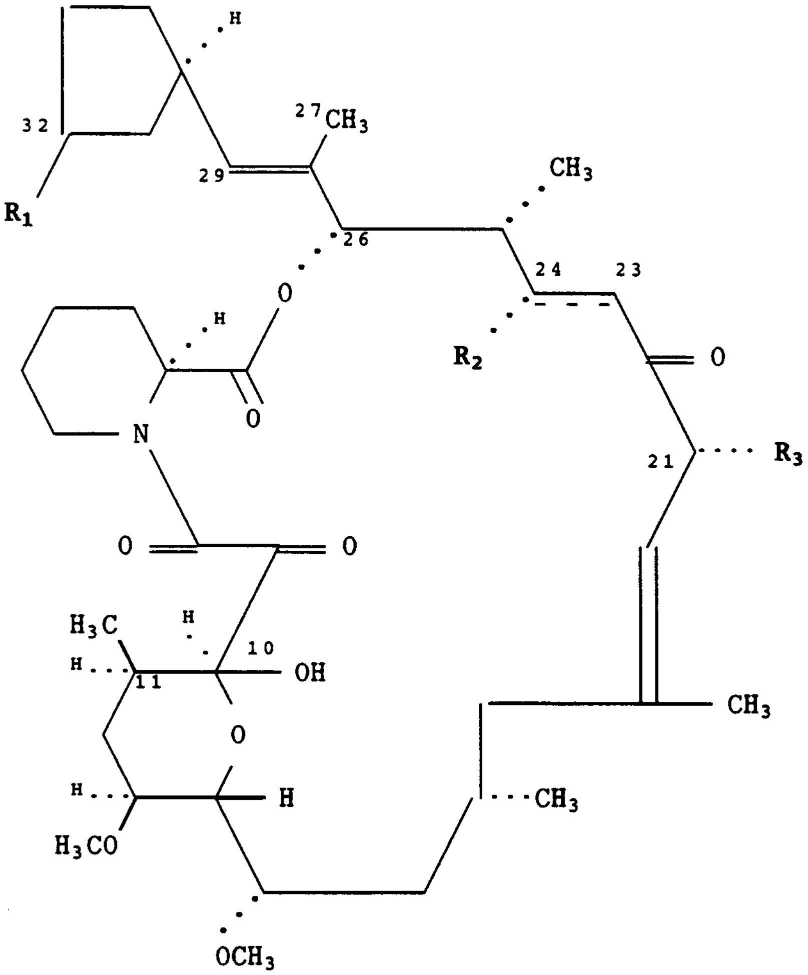

I

wherein the substituents have various significances. They are prepared by a process comprising ring contraction accompanied by appropriate derivatization. They possess interesting pharmacological activity as immunosuppressant and antiproliferative, antiinflammatory, and chemotherapeutic drug resistance reversing agents.

The invention relates to the field of macrolides. It concerns
a 12-(2′-cyclopentylvinyl)-11,28-dioxa-4-azatricyclo[22.3.1.$0^{4,9}$]octacos-18-ene or -14,18-diene
or an 11-(2′-cyclopentylvinyl)-10,27-dioxa-4-azatricyclo[21.3.1.$0^{4,8}$]heptacos-17-ene or -13,17-diene derivative,
with the proviso that the cyclopentyl moiety thereof is other than 3-formylcyclopentyl,
hereinafter referred to briefly as "a compound of the invention".

A preferred compound of the invention is
a 17-alkyl (or 17-alkenyl)-1-hydroxy-14-oxy-12-(2′-cyclopentyl-1′-methyl-trans-vinyl)-23,25-dimethoxy-13,19,21,27-tetramethyl-11,28-dioxa-4-azatricyclo[22.3.1.$0^{4,9}$]octacos-18- trans-ene-2,3,10,16-tetraone
or 17-alkyl (or 17-alkenyl)-1-hydroxy-12-(2′-cyclopentyl-1′-methyl-trans-vinyl)-23,25-dimethoxy-13,19,21,27-tetramethyl-11,28-dioxa-4-azatricyclo[22.3.1.$0^{4,9}$]octacos-14,18-trans- diene (or -18-trans-ene)-2,3,10,16-tetraone derivative,
with the proviso that the cyclopentyl moiety thereof is other than 3-formylcyclopentyl.

Even more preferred is
a 17α-alkyl (or 17α-alkenyl)-1β-hydroxy-14α-oxy-12-[2′-(cyclopent-1″(R)-yl)-1′-methyl-trans-vinyl]-23α,25β-dimethoxy-13α,19,21α,27β-tetramethyl-11,28-dioxa-4-azatricyclo [22.3.1.$0^{4,9}$]octacos-18-trans-ene-9αH,24βH-2,3,10,16-tetraone
or 17α-alkyl (or 17α-alkenyl)-1β-hydroxy-12-[2′-(cyclopent-1″(R)-yl)-1′-methyl-trans-vinyl]-23α,25β-dimethoxy-13α,19,21α,27β-tetramethyl-11,28-dioxa-4-azatricyclo [22.3.1.$0^{4,9}$]octacos-14,18-trans-diene (or -18-trans-ene)-9αH,24βH-2,3,10,16-tetraone derivative,
with the proviso that the cyclopentyl moiety thereof is other than 3-formylcyclopentyl.

The cyclopentyl moiety preferably is monosubstituted, preferably in the 3 position. The tricyclic ring structure preferably is substituted as is known from the literature, e.g. EP 184162, for analogous cyclohexyl tricyclic compounds.

A compound of the invention can be obtained by a process which comprises subjecting a suitable 12-(2′-cyclohexylvinyl)-11,28-dioxa-4-azatricyclo[22.3.1.$0^{4,9}$]octacos-18-ene or -14,18-diene or a suitable 11-(2′-cyclohexylvinyl)-10,27-dioxa-4-azatricyclo[22.3.1.$0^{4,8}$]heptacos-17-ene or -13,17-diene derivative to ring contraction and appropriately derivatizing the resultant cyclopentyl compound.

The invention includes further the use of this resultant cyclopentyl compound as an intermediate, e.g. in the preparation of a compound of the invention.

The process of the invention is effected in a manner analogous to known procedures. Ring contraction is e.g. effected by treating a corresponding derivative substituted in the cyclohexyl ring by a cyano group, with an acid having a non-nucleophilic anion, such as hydrofluoric acid. Preferably an inert solvent such as acetonitrile is used. The reaction is effected e.g. in a manner analogous to that described in EP 427680. Protecting groups which may conveniently be present can be simultaneously removed, or protecting groups may be introduced in conventional manner. The temperature preferably is about room temperature. The subsequent derivatization is also effected in conventional manner, e.g. according, or analogously to, the methods described hereunder.

The compounds of the invention may be isolated and purified from the reaction mixture in conventional manner.

The designations and numbering of carbon atoms used above are according to the designations and numbering in EP 184162 for analogous, cyclohexyl, compounds. However, various other designations and numbering are used in the literature. The numbering of carbon atoms used hereunder is in accordance with the numbering for compound FK506 published in H. Tanaka et al., J.Am.Chem.Soc. 109 (1987) 5031.

The invention concerns particularly the compounds of formula I

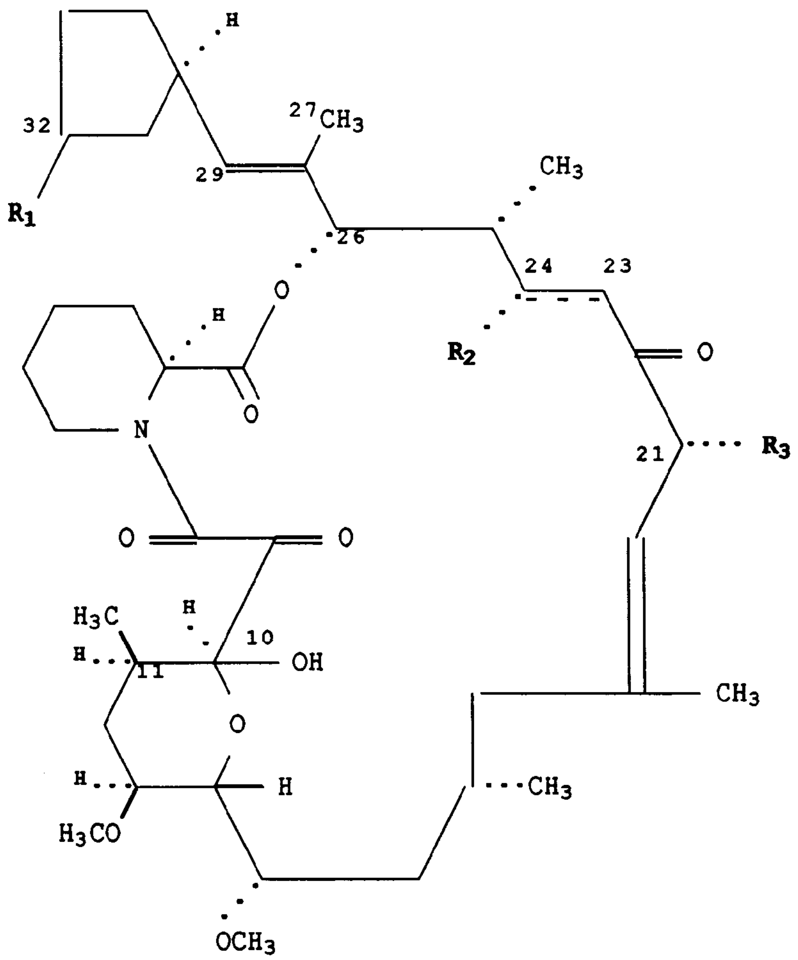

I

wherein

$R_1$ is methoxymethyl; optionally protected hydroxymethyl; acyloxymethyl wherein the acyl moiety optionally contains either a dimethylamino group which may optionally be quaternized, or a carboxy group which may optionally be esterified, or one or more amino and/or hydroxy groups which may optionally be protected; or aminooxalyloxymethyl;

either $R_2$ is methoxy, formyloxy or optionally protected hydroxy and there is a single bond between positions 23 and 24

or $R_2$ is absent and there is a double bond between positions 23 and 24; and

$R_3$ is methyl, ethyl, n-propyl or allyl;

in free form and, where such forms exist, in salt form,

hereinafter referred to briefly as "the particular compounds of the invention".

As appears from formula I the carbon atom in 32 position is asymmetrically substituted; $R_1$ can thus be bound with the $\alpha$ or the $\beta$ configuration to that carbon atom, and the compounds of formula I can exist as the corresponding individual isomers or as the diastereoisomeric mixture thereof.

A subgroup of particular compounds of the invention is the **compounds Ip1**, i.e. the compounds of formula I wherein

$R_1$ is hydroxymethyl;

either $R_2$ is optionally protected hydroxy and there is a single bond between positions 23 and 24

or $R_2$ is absent and there is a double bond between positions 23 and 24; and

$R_3$ is as defined above under formula I.

A further subgroup of particular compounds of the invention is the **compounds Ip2**, i.e. the compounds of formula I wherein

$R_1$ is optionally protected hydroxymethyl;

$R_2$ is as defined above for compounds $Ip_1$; and

$R_3$ is as defined above under formula I;

in free form and, where such forms exist, in salt form.

A further subgroup of particular compounds of the invention is the **compounds $Ip_3$**, i.e. the compounds of formula I wherein
$R_1$ is the acyloxymethyl group formyloxymethyl;
either $R_2$ is formyloxy or optionally protected hydroxy and there is a single bond between positions 23 and 24 or $R_2$ is absent and there is a double bond between positions 23 and 24; and
$R_3$ is as defined above under formula I,
in free form and, where such forms exist, in salt form.

A further subgroup of particular compounds of the invention is the **compounds $Ip_4$**, i.e. the compounds of formula I wherein
$R_1$ is acyloxymethyl wherein the acyl moiety optionally contains either a dimethylamino group which may optionally be quaternized, or one or more amino and/or hydroxy groups which may optionally be protected;
$R_2$ is as defined above for compounds $Ip_1$; and
$R_3$ is as defined above under formula I;
in free form and, where such forms exist, in salt form.

A further subgroup of particular compounds of the invention is the **compounds $Ip_5$**, i.e. the compounds of formula I wherein
$R_1$ is aminooxalyloxymethyl;
$R_2$ is as defined above for compounds $Ip_1$; and
$R_3$ is as defined above under formula I;
in free form and, where such forms exist, in salt form.

A further subgroup of particular compounds of the invention is the **compounds $Ip_6$**, i.e. the compounds of formula I wherein
either $R_1$ is methoxymethyl and
$R_2$ is methoxy or optionally protected hydroxy and there is a single bond between positions 23 and 24 or is absent and there is a double bond between positions 23 and 24;
or $R_1$ is optionally protected hydroxymethyl and
$R_2$ is methoxy; and
$R_3$ is as defined above under formula I;
in free form and, where such forms exist, in salt form.

In a suitable group acyloxymethyl the acyl moiety preferably is of altogether 1 to 20, preferably 1 to 5 carbon atoms, e.g. formyl, acetyl, isobutanoyl, benzoyl or pivaloyl. It is preferably formyl.

Suitable protecting groups for hydroxy and hydroxymethyl are conventional hydroxy-protecting groups such as tert-butoxycarbonyl or trialkylsilyl, preferably tert-butyldimethylsilyl.

Optionally protected hydroxymethyl or hydroxy as defined above under formula I for $R_1$ and $R_2$ should herein not be understood as including a group $R_1$ or $R_2$ which is specified elsewhere in the definitions for formula I, such as acyloxymethyl, aminooxalyloxymethyl, formyloxy or methoxy.

Protected amino preferably is amino protected by a conventional amino-protecting group such as benzyloxycarbonyl or trialkylsilyl; it especially is tert-butoxycarbonyl.

Esterified carboxy preferably is esterified with alkyl of 1 to 4 carbon atoms, it especially is methoxycarbonyl.

A "particular compound of the invention" preferably is in free form. It preferably is in unprotected form.

$R_1$ preferably is optionally protected hydroxymethyl. $R_2$ preferably is hydroxy. $R_3$ preferably is ethyl or allyl.

A further subgroup of particular compounds of the invention is the compounds of formula Is

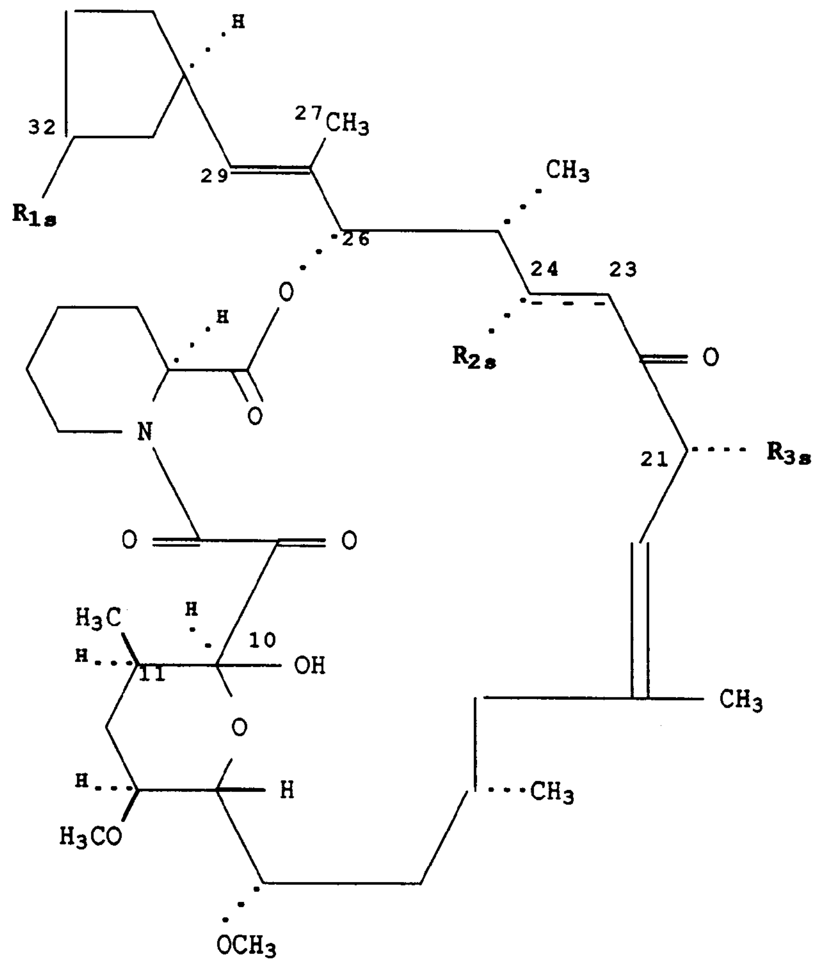

Is

wherein

$R_{1s}$ is methoxymethyl; hydroxymethyl optionally protected by tert-butyldimethylsilyl; benzoyloxymethyl; acyloxymethyl of 1 to 5 carbon atoms in the acyl moiety thereof, which optionally contains either a dimethylamino group which may optionally be quaternized or a carboxy group which may optionally be esterified by alkyl of 1 to 4 carbon atoms or an amino group which may optionally be protected by tert-butoxycarbonyl; or aminooxalyloxymethyl;

either $R_{2s}$ is methoxy, formyloxy or hydroxy optionally protected by tert-butyldimethylsilyl and there is a single bond between positions 23 and 24

or $R_{2s}$ is absent and there is a double bond between positions 23 and 24; and

$R_{3s}$ is ethyl or allyl,

in free form and, where such forms exist, in salt form.

The particular compounds of the invention can be obtained by a process comprising

a) for the preparation of a compound of formula I wherein

$R_1$ is hydroxymethyl,

either $R_2$ is optionally protected hydroxy and there is a single bond between positions 23 and 24

or $R_2$ is absent and there is a double bond between positions 23 and 24, and

$R_3$ is as defined above under formula I

(i.e. a **compound Ia**),

appropriately reducing a corresponding compound having a formyl group in position 32, i.e. a **compound II**, of formula II

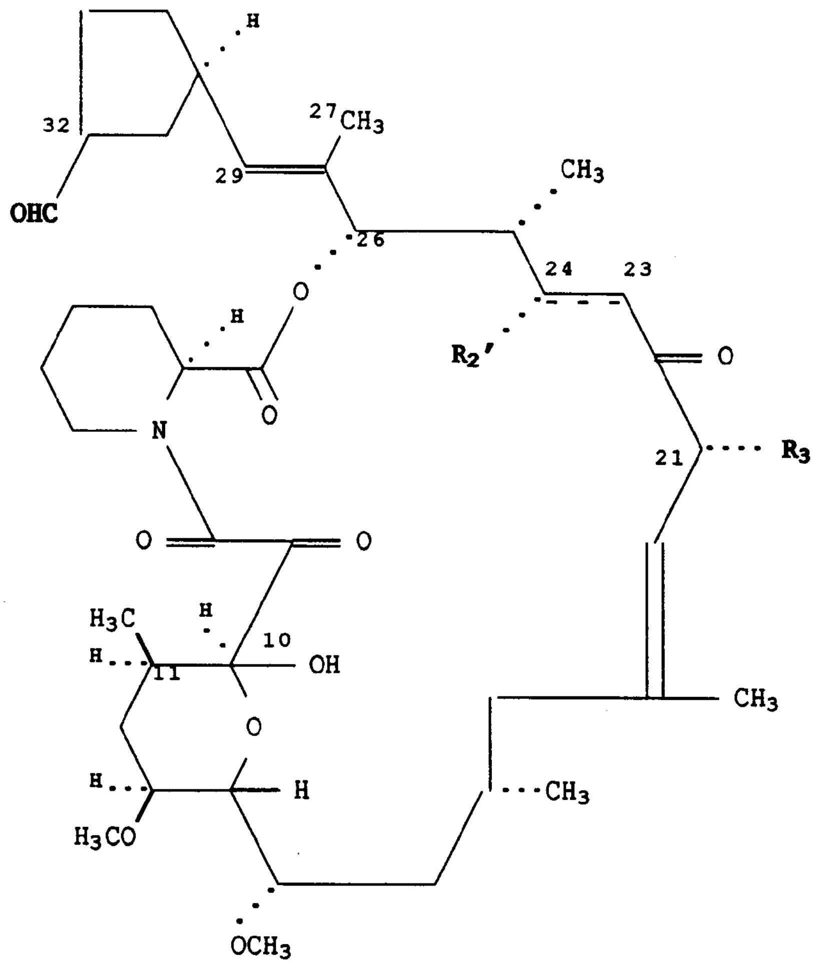

II

wherein

$R_2'$ either is optionally protected hydroxy and there is a single bond between positions 23 and 24, or is absent and there is a double bond between positions 23 and 24, and

$R_3$ is as defined above under formula I;

b) for the preparation of a compound of formula I wherein

$R_1$ is protected hydroxymethyl,

either $R_2$ is optionally protected hydroxy and there is a single bond between positions 23 and 24 or $R_2$ is absent and there is a double bond between positions 23 and 24, and

$R_3$ is as defined above under formula I

(i.e. a **compound Ib**),

appropriately introducing a protecting group into a compound Ia;

c) for the preparation of a compound of formula I wherein

$R_1$ is optionally protected hydroxymethyl or formyloxymethyl,

either $R_2$ is formyloxy or optionally protected hydroxy and there is a single bond between positions 23 and 24

or $R_2$ is absent and there is a double bond between positions 23 and 24, and

$R_3$ is as defined above under formula I,

with the proviso that at least one of $R_1$ and $R_2$ is formyloxymethyl or, respectively, formyloxy

(i.e. a **compound Ic**),

appropriately formylating a corresponding compound of formula I wherein $R_1$ is optionally protected hydroxymethyl or formyloxymethyl,

either $R_2$ is formyloxy or optionally protected hydroxy and there is a single between positions 23 and 24 or $R_2$ is absent and there is a double bond between positions 23 and 24, and

$R_3$ is as defined above under formula I,

with the proviso that at least one of $R_1$ and $R_2$ is other than formyloxymethyl or, respectively, formyloxy

(i.e. a **compound Id**);

d) for the preparation of a compound of formula I wherein
$R_1$ is aminooxalyloxymethyl,
either $R_2$ is optionally protected hydroxy and there is a single bond between positions 23 and 24
or $R_2$ is absent and there is a double bond between positions 23 and 24, and
$R_3$ is as defined above under formula I
(i.e. a **compound Ie**),
treating with an appropriate oxalyl derivative and thereafter with ammonia a corresponding compound Ia;
e) for the preparation of a compound of formula I wherein
$R_1$ is methoxymethyl or optionally protected hydroxymethyl,
either $R_2$ is methoxy or optionally protected hydroxy and there is a single bond between positions 23 and 24
or $R_2$ is absent and there is a double bond between positions 23 and 24, and
$R_3$ is as defined above under formula I,
with the proviso that at least one of $R_1$ and $R_2$ is methoxymethyl or, respectively, methoxy
(i.e. a **compound If**),
appropriately methylating a corresponding compound of formula I wherein
$R_1$ is optionally protected hydroxymethyl,
either $R_2$ is optionally protected hydroxy and there is a single bond between positions 23 and 24
or $R_2$ is absent and there is a double bond between positions 23 and 24, and
$R_3$ is as defined above under formula I
(i.e. a **compound Ig**)
f) for the preparation of a compound of formula I wherein
$R_1$ is acyloxymethyl wherein the acyl moiety optionally contains either a dimethylamino group which may optionally be quaternized or a carboxy group which may optionally be esterified or one or more optionally protected amino and/or hydroxy groups,
either $R_2$ is optionally protected hydroxy and there is a single bond between positions 23 and 24
or $R_2$ is absent and there is a double bond between positions 23 and 24, and
$R_3$ is as defined above under formula I
(i.e. a **compound Ih**),
appropriately acylating a corresponding compound Ia,
and if desired quaternizing a resultant compound Ih wherein $R_1$ contains a dimethylamino group;

and when a resultant compound of formula I has a protected hydroxy or hydroxymethyl and/or a protected amino group, optionally removing the protecting group(s) to give a corresponding compound of formula I having one or more unprotected hydroxy or hydroxymethyl and/or unprotected amino group(s) (i.e. a **compound Ii**);
and recovering the resultant compound of formula I in free form or, where such forms exist, in salt form.

The invention includes further the use of a compound of formula II as an intermediate, e.g. in the preparation of a compound of the invention as defined above, especially of a particular compound of the invention as defined above.

A compound of formula II [which is a 12-(2′-cyclopentylvinyl)-11,28-dioxa-4-azatricyclo[22.3.1.$0^{4,9}$]octacos-18-ene or -14,18-diene derivative according to the nomenclature in EP 184162] can be obtained by treating a compound of formula III

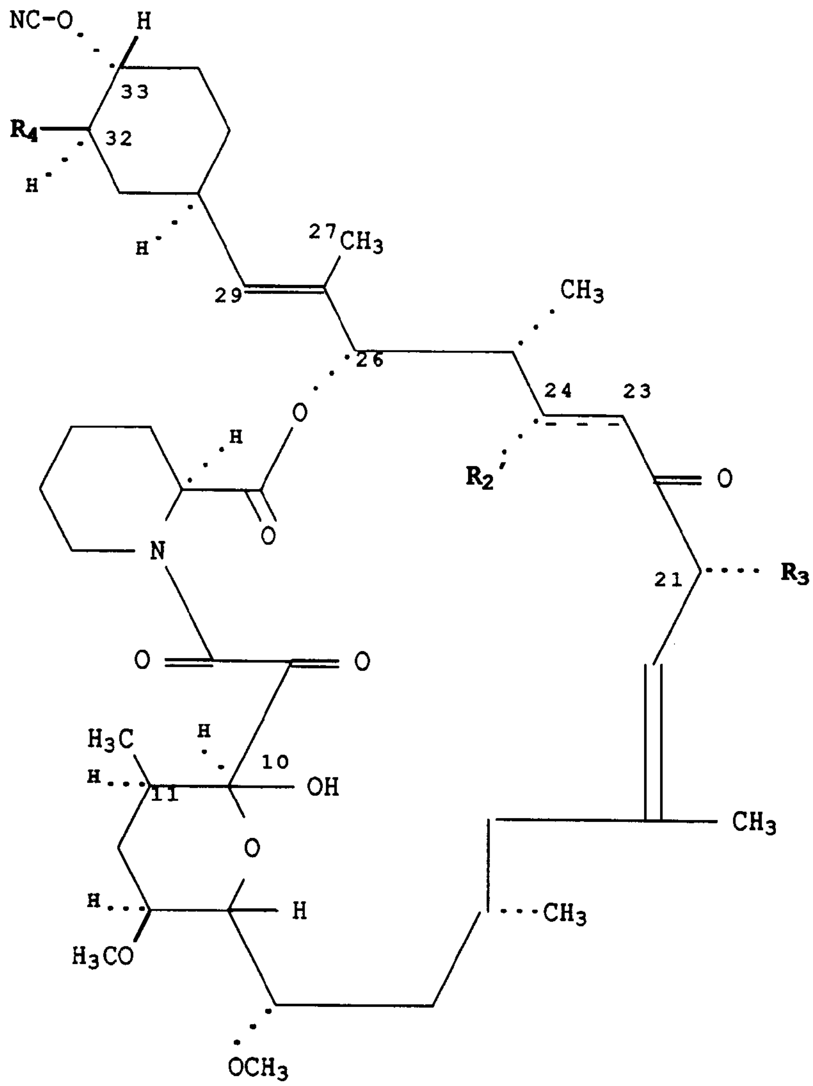

III

wherein
$R_4$ is hydroxy or methoxy,
$R_2'$ either is optionally protected hydroxy and there is a single bond between positions 23 and 24, or is absent and there is a double bond between positions 23 and 24, and
$R_3$ is as defined above under formula I
[which is a 12-(2'-cyclohexylvinyl)-11,28-dioxa-4-azatricyclo[22.3.1.0$^{4,9}$]-octacos-18-ene or -14,18-diene derivative according to the nomenclature in EP 184162] with an acid having a non-nucleophilic anion.

This reaction is a ring contraction. It is effected e.g. as described above.

It will be appreciated that process variants a) to f) and deprotection are thus illustrations of appropriate derivatization of the cyclopentyl compound obtained upon ring contraction.

A compound of formula III can be obtained e.g. as described in EP 427680 by treating a corresponding 33-hydroxy compound

– either with cyanogen bromide in the presence of a base;
– or with thiophosgene, reacting the resultant product with an inorganic azide and allowing the resultant unstable intermediate having a group

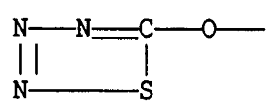

in 33 position to decompose to a corresponding compound of formula III.

Insofar as their preparation is not specifically described herein, e.g. in the Examples, the compounds used as starting materials are known or can be obtained in conventional manner from known compounds, e.g. starting from appropriate Streptomyces strains such as Streptomyces tsukubaensis No. 9993 described in e.g. Fujisawa EP 184162. Samples can be obtained from the Fermentation Research Institute, Tsukuba, Ibaraki 305, Japan under provisions of the Budapest Treaty under deposit No. FERM BP-927. This strain has been redeposited on April 27, 1989 with the Agricultural Research Culture Collection International Depository, Peoria, Illinois 61604, USA under the provisions of the Budapest Treaty under deposit No. NRRL 18488.

The process variants leading to the "particular compounds of the invention" can be effected in conventional manner.

Process variant **a)** is a reduction to the alcohol. It preferably is effected in an inert solvent, e.g. a cyclic ether. The reducing agent is e.g. lithium-B-isopinocamphenyl-2-borabicyclo [3.3.1]nonylhydride (R-Alpinhydride™, Aldrich) in a cyclicether such as tetrahydrofuran, or borane-tert-butylamine complex in a mixture of water and tetrahydrofuran. Temperature preferably is low, e.g. of from about -100°C to about -20°C with R-Alpinhydride™, preferably approximately -78°C, or of from about -50°C to about -20°C with borane tert-butylamine complex, preferably about 0°C. Reduction can alternatively be effected with diphenylsilane using a catalyst such as a Wilkinson catalyst, preferably in an inert solvent such as an aromatic hydrocarbon, e.g. benzene. Diastereoisomeric mixtures (position 32) may be obtained which can, if desired, be fractionated in conventional manner, e.g. chromatographically.

Process variant **b)** is an introduction of a protecting group. It may also be effected in conventional manner along similar lines. Thus for reactions involving a hydroxy group, particularly a hydroxy group in both position 24 and on the substituent in position 32, selective protection of only one of the two free hydroxy groups or selective deprotection of only one of the two protected hydroxy groups may be effected in such a manner that substitution occurs only at the desired position. Reaction may however also be effected with both positions unprotected. Mixtures of end products may be obtained thereby; such mixtures can be separated in conventional manner, e.g. chromatographically. Resultant end products still containing protecting groups can be subsequently deprotected, if desired. Reaction conditions may alternatively be selected such that simultaneously with or immediately after reaction the protecting groups are removed (one-pot process). Depending on the reaction conditions used, such as the duration of reaction, products are obtained which are predominantly monoprotected in the substituent in position 32 or diprotected in position 24 and in the substituent in position 32.

Process variant **c)** is a formylation. It is effected e.g. with a mixture of an oxalyl halogenide, e.g. the chloride, and dimethylformamide (this mixture in solution in e.g. acetonitrile gives a Vilsmeyer complex). The reaction is effected at room or slightly reduced temperature, e.g. at from about room temperature to about 5°C. Formylation may also be effected with the mixed anhydride of acetic and formic acid. A protecting group may be simultaneously removed.

Process variant **d)** is an acylation. It is preferably effected in an inert solvent such as acetonitrile. Temperature preferably is about room temperature or somewhat reduced, e.g. from about 25° to about 0°C. The oxalyl derivative preferably is an oxalyl halogenide, e.g. the chloride. Upon completion of the reaction the mixture is stirred with ammonia. Thereupon a double bond may be formed between positions 23 and 24.

Process variant **e)** is a methylation. It preferably is effected in an inert solvent such as a chlorinated hydrocarbon, e.g. dichloromethane. The methylating agent preferably is diazomethane in the presence of a Lewis acid, e.g. borontrifluoride-etherate. Temperature preferably is from about 0°C to about room temperature. A protecting group may be simultaneously removed.

Process variant **f)** is again an acylation. It preferably is effected in an inert solvent such as acetonitrile. The acylating agent preferably is an activated acyl derivative, such as an acyl halogenide or anhydride. An acid scavenger such as dimethylaminopyridine or pyridine is normally employed. Further, a carboxylic acid may be used, such as glycine protected at the amino moiety by e.g. tert-butoxycarbonyl, or a carbodiimide such as N-ethyl-N'-(3-dimethylaminopropyl)carbodiimide or N,N'-dicyclohexylcarbodiimide, where indicated in the presence of a base such as 4-dimethylaminopyridine, preferably in an inert solvent such as acetonitrile or in a chlorinated hydrocarbon. An amino protecting group may subsequently be removed together with any hydroxy protecting group which may be present. If in the starting compound Ia $R_2$ is hydroxy and there is a single bond between positions 23 and 24, upon acylation elimination of a water molecule in 23,24 position may occur and a final compound be formed wherein $R_2$ is absent and there is a double bond between positions 23 and 24.

The optional subsequent quaternization step is also effected in conventional manner, e.g. by treatment with an alkyl iodide, preferably methyl iodide, in an inert solvent such as acetone.

The optional **deprotection** step may also be effected in conventional manner. For removal of e.g. tert-butyldimethylsilyl or tert-butoxycarbonyl it may be effected by treatment with hydrofluoric acid in a solvent such as acetonitrile. Depending on the reaction conditions chosen (e.g. duration or temperature) the removal can be steered in such a manner that either all or only some protecting groups are eliminated. Partial deprotection is

particularly indicated where a definite hydroxy group is to be reacted in a subsequent reaction.

The following Examples illustrate the invention. They are not limitative. All temperatures are in degrees Centigrade. In the NMR spectra all chemical shift values are in ppm; samples are measured in $CDCl_3$. The following abbreviations are used:

| | |
|---|---|
| BOC: | tert-butoxycarbonyl; |
| cfr: | colourless foamy resin: |
| db: | double bond; |
| OMe (or MeO): | methoxy; |
| OtBDMS: | tert-butyldimethylsilyloxy; |
| sb: | single bond; |
| FK 506: | the compound of formula |

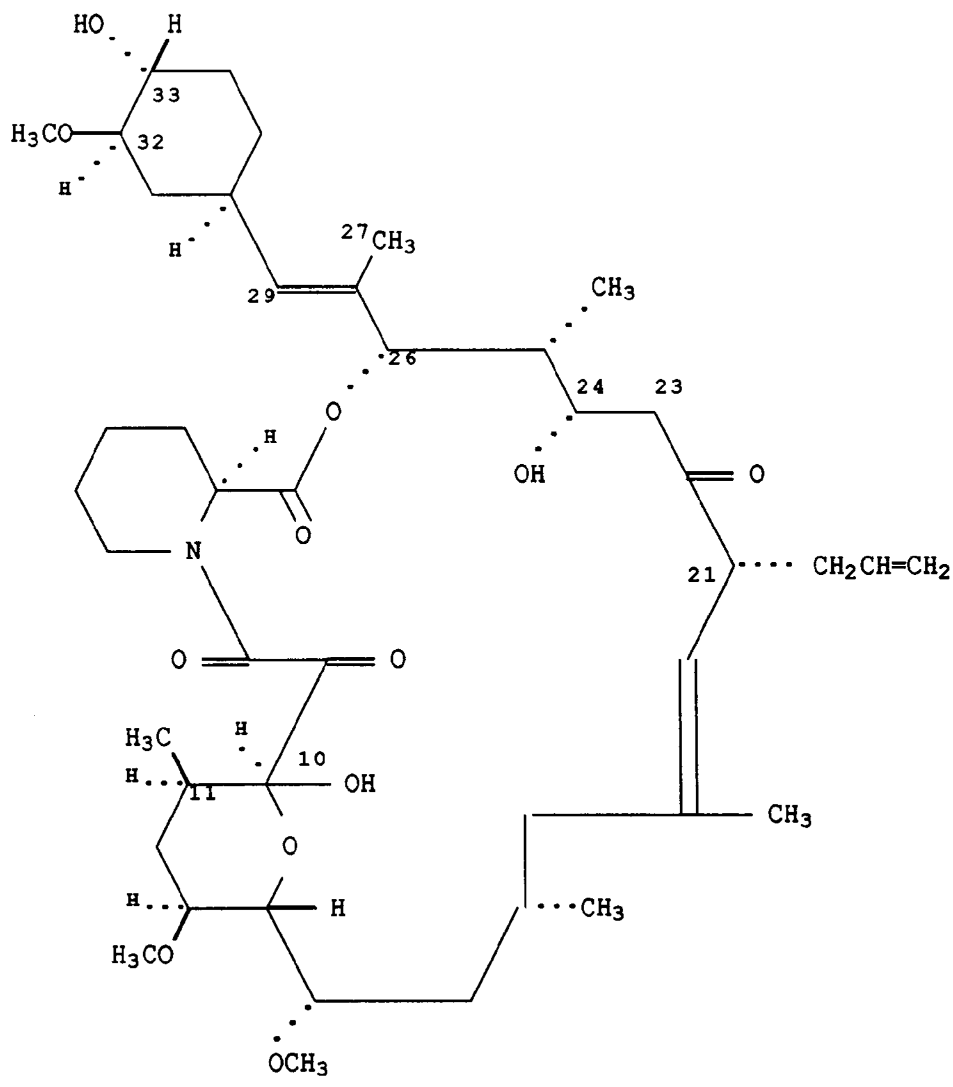

i.e. 17α-allyl-1β,14α-dihydroxy-12-[2′-(4″(R)-hydroxy-3″(R)-methoxycyclohex-1″(R)-yl)-1′-methyl-trans-vinyl]-23α,25β-dimethoxy-13α,19,21α,27β-tetramethyl-11,28- dioxa-4-azatricyclo[22.3.1.0$^{4,9}$]octacos-18-trans-ene-2,3,10,16-tetraone

(according to the atom numbering in EP 184162; however, in the Examples the atom numbering of formula I is used throughout);

FR 520: as FK 506, but with ···$CH_2CH_3$ (ethyl) in place of allyl in position 21 in the formula;

cyclopentyl-FK506: the compound of formula

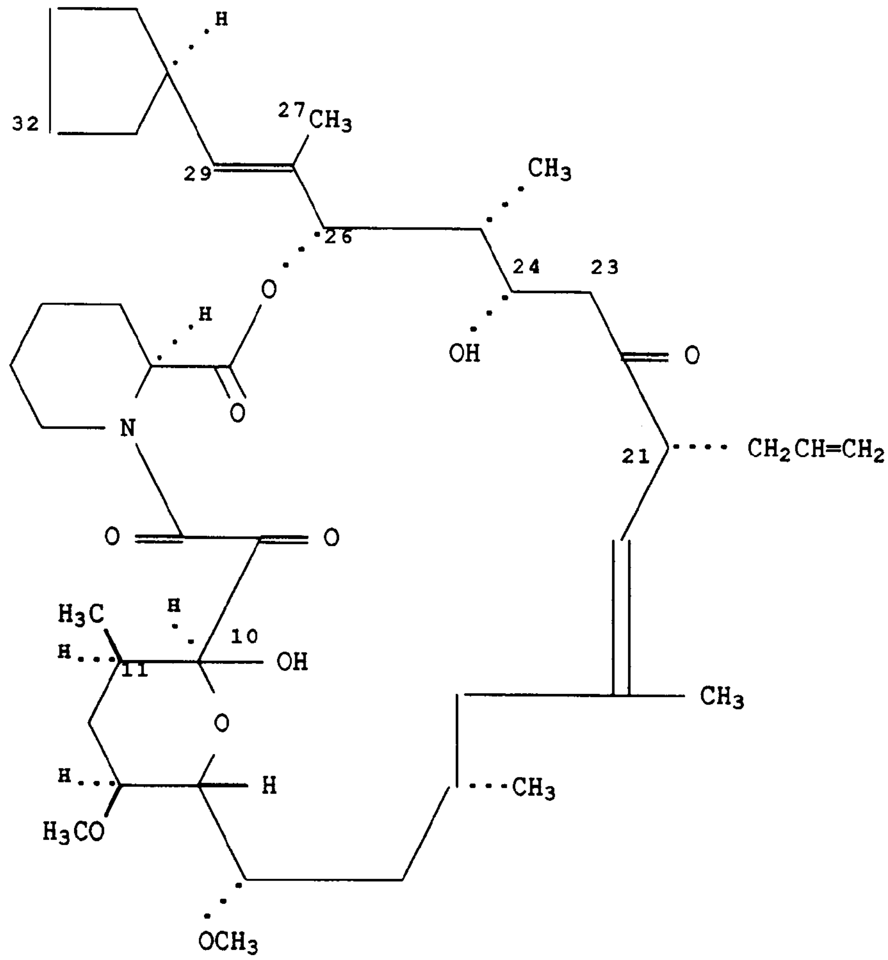

cyclopentyl-FR520: as cyclopentyl-FK506, but with ···$CH_2CH_3$ (ethyl) in place of allyl in position 21 in the formula.

**Example 1: 32-Hydroxymethyl-cyclopentyl-FR520**

[Formula I: $R_1$ = hydroxymethyl; $R_2$ = hydroxy, single bond between positions 23 and 24; $R_3$ = ethyl]

[Process variant a), reduction with a Wilkinson catalyst]

A stirred solution of 26 g **32-formyl-cyclopentyl-FR520** (compound of formula II; Example 12 in EP 427 680) in 150 ml of benzene is cooled to 15° and treated successively with 7.25 ml of diphenylsilane and thereafter with 200 mg of tris(phenylphosphine) rhodium (I) chloride and the cooling bath is removed immediately thereafter. The resultant exothermic reaction occurs under increasing gas formation and temperature rises to 29°. 40 minutes after addition the reaction mixture is extensively concentrated at a bath temperature not above 40° and a pressure of 30-40 mbar. The residue is taken up in 150 ml of acetonitrile, 5 ml of 40 % (w/w) aqueous hydrofluoric acid solution are added over 5 minutes and the mixture is stirred further for 25 minutes. The reaction mixture is poured onto a well stirred mixture of 850 ml of ethyl acetate and 500 ml of saturated aqueous sodium bicarbonate solution and the resultant solution is thoroughly mixed. The aqueous phase is separated and extracted with 100 ml of ethyl acetate. The combined organic phases are successively washed with 100 ml of saturated aqueous sodium chloride solution and twice with 100 ml portions of saturated aqueous sodium chloride solution, dried over sodium sulfate, filtered and the solvent is evaporated under reduced pressure. The residue is subjected to column chromatography (eluant: ethyl acetate / hexane 1:1) and the **title compound** is obtained as a diastereoisomeric mixture (cfr):
Diastereoisomer A:
$^1$H-NMR (approximately 2:1 mixture of conformers):
main conformer: 4.258 (d, J = 1 Hz, 10-OH);

minor conformer: 4.813 (d, J = 1.5 Hz, 10-OH);
$^{13}C$-NMR (approximately 2:1 mixture of conformers):
main conformer: 213.4 (C-22); 196.2 (C-9); 169.05 (C-1); 164.8 (C-8); 97.1 (C-10);
Diastereoisomer B:
1H-NMR (approximately 2:1 mixture of conformers):
main conformer: 4.297 (d, J = 1 Hz, 10-OH);
minor conformer: 4.795 (d, J = 1 Hz, 10-OH);
$^{13}C$-NMR (approximately 2:1 mixture of conformers):
main conformer: 213.4 (C-22); 196.12 (C-9); 169.05 (C-1); 164.74 (C-8); 97.13 (C-10).

The starting material is obtained as follows:

a) A solution of 2 g **24-O-tert-butyldimethylsilyl-FR520** and 0.94 g 4-dimethylaminopyridine in 100 ml of dichloromethane is rapidly reacted at room temperature with a solution of 0.4 g cyanogen bromide in 15 ml of dichloromethane and the mixture is stirred at room temperature for 20 minutes. The mixture is filtered over silicagel (eluant: n-hexane/acetic acid ethyl ester) and the solvent is removed from the relevant fraction under reduced pressure. **24-O-tert-Butyldimethylsilyl-33-O-cyano-FR 520** is obtained as a colourless foamy resin:
$^{1}H$-NMR: mixture of rotamers: 4.3 (m; H-33);

b) 0.5 g of the compound obtained under a) above is dissolved into a mixture of 50 ml of acetonitrile and 2 ml of 40 % wt. aqueous hydrofluoric acid and the mixture is stirred for 2.5 hours at room temperature. The reaction mixture is then partitioned between acetic ethyl acetate and saturated aqueous sodium bicarbonate solution, the aqueous phase is discarded and the organic phase is washed with saturated aqueous sodium chloride solution, dried over sodium sulfate, filtered and concentrated under reduced pressure. **32-Formyl-cyclopentyl-FR 520** is obtained as a colourless foamy resin from the residue by column chromatography over silicagel (eluant: n-hexane/ethyl acetate):
$^{1}H$-NMR: mixture of conformers: 9.64 (d, J=2 Hz, CHO); 2.87 (m, H-32); 2.67 (m, H-30).

## Example 2: 24-O-tert-Butyldimethylsilyl-32-hydroxymethyl-cyclopentyl-FR 520

[Formula I: $R_1$ = hydroxymethyl; $R_2$ = OtBDMS, single bond between positions 23 and 24; $R_3$ = ethyl]

[Process variant a), reduction with R-Alpinhydride™ or borane-tert-butylamine complex]

A solution of 180 mg **24-O-tert-butyl-dimethylsilyl-32-formyl-cyclopentyl-FR 520** (compound of formula II; Example 71 in EP 427680) in 15 ml of tetrahydrofuran is treated at -78° with 0.5 ml of a 0.5 M solution of lithium-B-isopinocamphenyl-2-borabicyclo[3.3.1]nonylhydride (R-Alpinhydride™, Aldrich) in tetrahydrofuran and the mixture is stirred for 1 hour at that temperature. The reaction mixture is partitioned between 1 N HCl and ethyl acetate, the organic phase is separated, washed with 1 N HCl and saturated aqueous sodium chloride solution, dried over sodium sulfate, filtered and concentrated under reduced pressure. The residue is chromatographed by column chromatography over silicagel (eluant: n-hexane/ethyl acetate 1.5:1). The **title compound** is obtained (cfr).

In a **variant** the reaction is effected in 40 ml of tetrahydrofuran and 10 ml of water at 0-5° with 1 molar equivalent of borane-tert-butyl-amine complex. Stirring is maintained for 5 minutes at 0-5°, the reaction mixture is added to a mixture of 100 ml of 1 N hydrochloric acid and 150 ml of ethyl acetate and stirred for 15 minutes, the organic phase is separated and working up effected as above. The **title compound** is obtained:
$^{1}H$-NMR: 5.42 (d, J = 9 Hz, H-29); 5.21 (d, J = 7.5 Hz; H-26); 4.81 (d, J = 10 Hz, H-20); 3.82 (dxd, J = 9 and 1 Hz; H-14); 3.51 (m, $CH_2OH$).

The starting material is obtained as follows:

A solution of 0.5 g **32-formyl-cyclopentyl-FR 520** [compound obtained under b) in Example 1], 0.5 g tert-butyl-dimethylsilyl chloride and 0.25 g imidazole in 20 ml of dry dimethylformamide is stirred for 15 hours at room temperature and thereafter partitioned between water and ethyl acetate. The organic phase is separated, washed with saturated aqueous sodium chloride solution, dried over sodium sulfate, filtered and concentrated under reduced pressure. **24-O-tert-Butyldimethylsilyl-32-formyl-cyclopentyl-FR 520** is obtained from the residue as a colourless foamy resin following column chromatography over silicagel (eluant: n-hexane/ethyl acetate 2:1):
$^{1}H$-NMR: mixture of rotamers: 9.65 (d, J = 2 Hz, CHO); 5.39 (d, J = 9 Hz, H-29); 5.01 (d, J = 7.5 Hz, H-26); 4.81 (d, J = 10 Hz, H-20); 3.82 (dxd, J = 9/2 Hz, H-14).

**Example 3**: **32-tert-Butyldimethylsilyloxymethyl-cyclopentyl-FR 520**

[Formula I: $R_1$ = $CH_2OtBDMS$; $R_2$ = hydroxy, single bond between positions 23 and 24; $R_3$ = ethyl]

[Process variant b), protection]

A solution of 0.5 g **32-hydroxymethyl-cyclopentyl-FR520** (compound of Example 1) in 30 ml of dimethylformamide is treated with 3 molar equivalents of **tert-butyldimethylsilyl chloride** and 3 molar equivalents of imidazole and the mixture is stirred for 2.5 hours at room temperature. The resultant mixture is partitioned between ethyl acetate and water, the organic phase is decanted, dried over sodium sulfate, filtered and concentrated. The **title compound** (cfr) is obtained from the residue by column chromatography over silicagel (eluant: n-hexane / ethyl acetate 3:1):
$^1H$-NMR:
Diastereoisomer A: 5.34 (d, J = 2.5 Hz, H-26); 4.6 (db, J = 4 Hz, H-2); 4.44 (db, J = 13 Hz, H-6 equ.); 4.25 (sb, 10-OH); 3.92 (m, H-24); 3.69 (d, J = 9 Hz, H-14); 3.59 (dxd, J = 2.5 and 10 Hz, H-15); 3.465 (d, J = 6 Hz, $-CH_2OSi$); 3.40 and 3.31 (s and s, 2x-$OCH_3$); 0.9 (s, t-butyl); 0.04 (s, Si-$CH_3$).
Diastereoisomer B: 5.34 (d, J = 2.5 Hz, H-26); 4.6 (db, J = 4 Hz, H-2); 4.44 (db, J = 13 Hz, H-6 equ.); 4.20 (sb, 10-OH); 3.92 (m, H-24); 3.69 (d, J = 9 Hz, H-14); 3.59 (dxd, J = 2.5 and 10 Hz, H-15); 3.465 (d, J = 6 Hz, $-CH_2OSi$); 3.40 and 3.31 (s and s, 2x-$OCH_3$); 0.9 (s, t-butyl); 0.04 (s, Si-$CH_3$).

**Example 4**: **24-O-Formyl-32-formyloxymethyl-cyclopentyl-FR 520**

[Formula I: $R_1$ = formyloxymethyl; $R_2$ = formyloxy, single bond between positions 23 and 24; $R_3$ = ethyl]

[Process variant c), formylation]

A solution of 0.5 g **32-hydroxymethyl-cyclopentyl-FR 520** (compound of Example 1) in 30 ml of acetonitrile is treated with 0.5 g 4-dimethylaminopyridine and 0.3 ml of mixed formic/acetic anhydride and stirred at room temperature for 30 minutes. The mixture is partitioned between ethyl acetate and 1 N hydrochloric acid, the organic phase is decanted, washed with saturated aqueous sodium chloride solution, filtered and concentrated under reduced pressure. The **title compound** (cfr) is obtained from the residue by column chromatography over silicagel (eluant: n-hexane/ethyl acetate 2:1):
$^1H$-NMR: mixture of conformers: 8.08 and 8.01 (s and s, 2x-O-CHO); 4.90 (d, J = 9 Hz, H-20); 4.55 (m, H-2); 4.44 (db, J = 13 Hz, H-6 equ.); 4.05 (d, J = 7.5 Hz, $-CH_2$-OCO-); 3.77 (dxd, J = 9 and 1 Hz, H-14).

**Example 5**: **a) 32-Aminooxalyloxymethyl-24-dehydroxy-$\Delta^{23}$-cyclopentyl-FR 520** and **b) 32-Aminooxalyloxymethyl-cyclopentyl-FR 520**

[Formula I: $R_1$ = aminooxalyloxymethyl; $R_2$ = absent, double bond between positions 23 and 24 and, respectively, $R_2$ = hydroxy, single bond between positions 23 and 24; $R_3$ = ethyl]

[Process variant d), treatment with oxalyl derivative and thereafter ammonia]

0.5 g **24-O-tert-Butyldimethylsilyl-32-tert-butyldimethylsilyloxymethyl-cyclopentyl-FR 520** (compound of Example 16) in 30 ml of acetonitrile is treated with 0.2 ml of oxalyl chloride and the mixture is stirred for 3 hours at room temperature. The reaction mixture is then added to a vigourously agitated mixture of 200 ml of ethyl acetate, 20 ml of saturated aqueous ammonia solution and 100 ml of saturated aqueous sodium chloride solution and the solution stirred for 20 minutes. Undissolved components are filtered off, the organic phase is decanted, successively washed with water, 1 N hydrochloric acid and water, dried over sodium sulfate, filtered and concentrated under reduced pressure. The residue is taken up in 30 ml of acetonitrile, treated with 1.5 ml of 40 % w/w aqueous hydrofluoric acid and the solution is stirred for 3 hours at room temperature. The solution is partitioned between ethyl acetate and saturated aqueous sodium bicarbonate solution, the organic phase is decanted, dried over sodium sulfate, filtered and concentrated. The **title compounds** (cfr) are obtained from the residue by column chromatography over silicagel (eluant: n-hexane/ethyl acetate 2:1):
$^1H$-NMR:
Compound a): mixture of conformers:

6.97 and 5.77 (b, $-CONH_2$); 6.83 (dxd, J = 6 and 15 Hz, H-24); 6.18 (dxd, J = 1 and 15 Hz, H-23); 4.20 (m, $-CH_2OC=O$);
Compound b): mixture of conformers:
6.97 and 5.92 (b, $-CONH_2$); 4.60 (d, J = 3 Hz, H-2); 4.44 (db, J = 13 Hz, H-6 equ.); 4.19 (m, $-CH_2OC=O$); 3.92 (m, H-24); 3.68 (d, J = 9 Hz, H-14); 3.40 and 3.32 (2x s, $2x-OCH_3$).

**Example 6: 24-Methoxy-32-methoxymethyl-cyclopentyl-FR 520**

[Formula I: $R_1$ = methoxymethyl; $R_2$ = methoxy, single bond between positions 23 and 24; $R_3$ = ethyl]

[Process variant e), methylation]

A solution of 1 g **32-hydroxymethyl-cyclopentyl-FR 520** (compound of Example 1) and 0.04 ml of borotrifluoride etherate in 50 ml of dichloromethane is cooled to 0-5° and treated with portions of a 10 ml solution (approximately 0.5 M) of diazomethane in dichloromethane in such a manner that the initial yellow coloration which forms upon addition persists for as shortly as possible. The mixture is then partitioned between ethyl acetate and saturated aqueous sodium bicarbonate solution, the organic phase is decanted, dried over sodium sulfate, filtered and concentrated. The **title compound** (cfr) is obtained from the residue by column chromatography over silicagel (eluant: n-hexane/ethyl acetate 2:1):
$^1H$-NMR: mixture of conformers:
5.37 (d, J = 8.8 Hz; H-29); 5.18 (d, J = 7.6 Hz, H-26); 4.77 (d, J = 10.2 Hz, H-20); 3.81 (dxd, J = 9.7/1 Hz, H-14); 2.67 (dxd, J = 13.5/8.4 Hz, H-23).

**Example 7: 24-O-tert-Butyldimethylsilyl-32-isobutanoyloxymethyl-cyclopentyl-FR 520**

[Formula I: $R_1$ = isobutanoyloxymethyl; $R_2$ = OtBDMS, single bond between positions 23 and 24; $R_3$ = ethyl]

[Process variant f), acylation]

0.5 g **24-O-tert-butyldimethylsilyl-32-hydroxymethyl-cyclopentyl-FR 520** (compound of Example 2) and 0.5 g dimethylaminopyridine in 30 ml of acetonitrile are treated with 0.3 ml of isobutyric acid anhydride and the mixture is stirred for 4 hours at room temperature. The mixture is then partitioned between ethyl acetate and 1 N aqueous hydrochloric acid solution, the organic phase is decanted, washed with saturated aqueous sodium chloride solution, filtered and concentrated under reduced pressure The **title compound** (cfr) is obtained from the residue by column chromatography over silicagel (eluant: n-hexane/ethyl acetate 3:1):
$^1H$-NMR: mixture of conformers
3.95 (d, J = 7 Hz, $-CH_2OCO-$); 2.55 [sept, J = 7 Hz, $H-C(CH_3)_2$]; 1.17 (d, J = 7 Hz, $2x-CH_3$).

**Example 8: 24-O-tert-Butyldimethylsilyl-32-[N,N-dimethylamino)methyl]-carbonyloxymethyl-cyclopentyl-FR 520**

[Formula I: $R_1$ = [(N,N-dimethylamino)methyl]carbonyloxymethyl; $R_2$ = OtBDMS, single bond between positions 23 and 24; $R_3$ = ethyl]

[Process variant f), acylation]

A solution of 1 g **24-O-tert-butyldimethylsilyl-32-hydroxymethyl-cyclopentyl-FR 520** (compound of Example 2) in 35 ml of absolute pyridine is treated with 1.2 equivalent of N,N'-dicyclohexylcarbodiimide, 1.2 equivalent of **N,N-dimethylglycine** and 0.2 equivalent of p-toluenesulfonic acid monohydrate and the mixture is stirred for 15 hours at room temperature. The reaction mixture is then partitioned between water and ethyl acetate, the organic phase is decanted, successively washed with 1 N hydrochloric acid solution and saturated aqueous sodium chloride solution, dried over sodium sulfate, filtered and concentrated under reduced pressure. The **title compound** (cfr) is obtained from the residue by a column chromatography over silicagel (eluant: ethyl acetate/methanol 20:1):
Characterization data: deprotection gives the compound of Example 20.

**Example 9: 32-[N-tert-Butoxycarbonylamino)methyl]carbonyloxymethyl-24-O-tert-butyldimethylsilyl-cyclopentyl-FR 520**

[Formula I: $R_1$ = BOC-$NHCH_2COOCH_2$-; $R_2$ = OtBDMS, single bond between positions 23 and 24; $R_3$ = ethyl]

[Process variant f), acylation]

A solution of 0.5 g **24-O-tert-butyldimethylsilyl-32-hydroxy-methyl-cyclopentyl-FR 520** (compound of Example 2), 0.3 g N-BOC-glycine and 0.5 g dicyclohexylcarbodiimide in 30 ml of acetonitrile is stirred at room temperature for 8 hours. The mixture is then diluted with 30 ml of ether and insoluble compounds are filtered off. The solution is then partitioned between ethyl acetate and aqueous 1 N hydrochloric acid solution, the organic phase is decanted, washed with saturated aqueous sodium chloride solution, filtered, dried over sodium sulfate and concentrated under reduced pressure. The **title compound** (cfr) is obtained from the residue by column chromatography over silicagel (eluant: n-hexane/ethyl acetate 3:1):
$^1$H-NMR: mixture of conformers:
4.81 (d, J = 10 Hz, H-20); 4.44 (H-2); 4.42 (H-6 equ.); 4.03 (d, J = 7 Hz, -$CH_2$OC=O); 3.91 (d, J = 6 Hz, -NH-$CH_2$C=O); 3.82 (dxd, J = 1.5 and 9 Hz, H-14); 3.40 and 3.32 (s and s, 2x-$OCH_3$); 1.46 (s, t-butyl-OC=O); 0.87 (s, t-butyl-Si).

**Example 10: 32-[N,N,N-Trimethylammoniomethyl)carbonyloxymethyl-cyclopentyl-FR 520 iodide**

[Formula I: $R_1$ = $(H_3C)_3N^+CH_2COOCH_2$-; $R_2$ = OH, single bond between positions 23 and 24; $R_3$ = ethyl; in iodide quaternary ammonium salt form]

[Process variant f): optional quaternization]

A solution of 0.5 g **32-(N,N-dimethyaminomethyl)carbonyloxymethyl-cyclopentyl-FR 520** (compound of Example 20) in 25 ml of acetone and 10 ml of diethylether is treated at room temperature and in darkness with 0.5 ml of methyl iodide and the solution is stirred for 8 hours. Then the reaction mixture is diluted with several portions of diethyl ether, the precipitate is collected by suction, repeatedly washed with diethylether and dried under high vaccum. The **title compound** is obtained (light yellow amorphous powder):
$^1$H-NMR: mixture of conformers:
3.66 (s, $N(CH_3)_3^+$); 4.99 (sb, N-$CH_2$-CO).

**Example 11: 32-Isobutanoyloxymethyl-cyclopentyl-FR 520**

[Formula I: $R_1$ = isobutanoyloxymethyl; $R_2$ = OH, single bond between positions 23 and 24; $R_3$ = ethyl)

[Process variant: deprotection]

A solution of 0.8 g **24-O-tert-butyldimethylsilyl-32-isobutanoyl-oxymethyl-cyclopentyl-FR 520** (compound of Example 7) in 40 ml of acetonitrile is treated with 2.5 ml of 40 % w/w hydrofluoric acid solution and the mixture is stirred for 6 hours at room temperature. The reaction mixture is then partitioned between ethyl acetate and saturated aqueous sodium bicarbonate solution, the organic phase is decanted, dried over sodium sulfate, filtered and concentrated. The **title compound** (cfr) is obtained from the residue by column chromatography over silicagel (eluant: n-hexane/ethyl acetate 2:1):
$^1$H-NMR: See Example 21.

The following compounds of formula I are obtained in analogous manner:

| Example No. | Analogously to Example No. | $R_1$ | $R_2$ | Position 23,24 | $R_3$ | Process variant | Physicochemical characterization data |
|---|---|---|---|---|---|---|---|
| 12 | 2;11[12)] | hydroxymethyl | OH | sb | ethyl | a); depr. | NMR*; NMR** |
| 13 | 1;11[15)] | hydroxymethyl | OtBDMS | sb | ethyl | a); depr. | NMR* |
| 14 | 1[1)];2[1)] | hydroxymethyl | absent | db | ethyl | a) | NMR* |
| 15 | 1[2)];2[2)] | hydroxymethyl | OH | sb | allyl | a) | NMR* |
| 16 | 5[3)] | $-CH_2OtBDMS$ | OtBDMS | sb | ethyl | b) | cfr; NMR* |
| 17 | 7[5)] | benzoyloxymethyl | OtBDMS | sb | ethyl | f) | cfr; NMR* |
| 18 | 7[5)] | acetoxymethyl | OtBDMS | sb | ethyl | f) | cfr; NMR* |
| 19 | 7[5)] | pivaloyloxymethyl | OtBDMS | sb | ethyl | f) | cfr; NMR* |
| 20 | 8[4)];11[11)] | $(H_3C)_2NCH_2COOCH_2-$ | OH | sb | ethyl | f); depr. | cfr; NMR* |
| 21 | 7[4)];11[13)] | isobutanoyloxymethyl | OH | sb | ethyl | f) | cfr; NMR* |
| 22 | 7[4)];11[7)] | benzoyloxymethyl | OH | sb | ethyl | f); depr. | cfr; NMR* |
| 23 | 7[4)];11[8)] | acetoxymethyl | OH | sb | ethyl | f); depr. | cfr; NMR* |
| 24 | 7[4)];11[9)] | pivaloyloxymethyl | OH | sb | ethyl | f); depr. | cfr; NMR* |
| 25 | 9[4)] | $BOC-NHCH_2COOCH_2-$ | OH | sb | ethyl | f) | cfr; deriv.*** |
| 26 | 7[5)] | $HOOC(CH_2)_2COOCH_2-$ | OtBDMS | sb | ethyl | f) | cfr; NMR* |
| 27 | 7[5)] | $H_3COOC(CH_2)_2COOCH_2-$ | OtBDMS | sb | ethyl | f) | cfr; deriv.*** |
| 28 | 7[4)];11[14)] | $HOOC(CH_2)_2COOCH_2-$ | OH | sb | ethyl | f); depr. | cfr; NMR* |
| 29 | 11[6)] | $H_3COOC(CH_2)_2COOCH_2-$ | OH | sb | ethyl | depr. | cfr; NMR** |
| 30 | 11[10)] | $H_2NCH_2COOCH_2-$ | OH | sb | ethyl | depr. | cfr; NMR* |

1) Starting from the compound of Example 14 in EP 427680;
2) Starting from the compound of Example 13 in EP 427680;
3) Starting from the compound of Example 2; or starting from the compound of Example 1 or 3 using 5 molar equivalents of tert-butyldimethylsilyl chloride and of imidazole and reacting for 15 hours;
4) Starting from the compound of Example 1;
5) Starting from the compound of Example 2;
6) Starting from the compound of Example 27;
7) Starting from the compound of Example 17;
8) Starting from the compound of Example 18;
9) Starting from the compound of Example 19;
10) Starting from the compounds of Examples 9 and 25
11) Starting from the compound of Example 8;
12) Starting from the compounds of Examples 2, 3, 4, 13 and 16;
13) Starting from the compound of Example 7;
14) Starting from the compound of Example 26;
15) Starting from the compound of Example 16;

*** Removal of the BOC or tert-butyldimethylsilyl protecting group gives the compound of Example 30 and, respectively, Example 29;

** $^{13}$C-NMR: Example 12: see under Example 1;

Example 29: mixture of rotamers:
main component: 213.5 (C-22); 196.19 (C-9); 164.75 (C-8); 172.4 and 169.06 (C=O, succinate); 97.04 (C-10);

* $^1$H-NMR:

Example 12: see under Example 1;

Example 13: see under Example 2;

Example 14: mixture of conformers; main conformer: 6.84 (dxd, J = 16 and 6 Hz, H-24); 6.19 (dxd, J = 16 and 2 Hz, H-23); 5.08 (d, J = 10 Hz, H-29); 4.41 (db, J = 13 Hz, H-6e); 4.30 (db, J = 5 Hz, H-2);

Example 15: approximately 7:3 mixture of conformers; main conformer: 5.72 (m, H-37); 5.326 (d, J = 2.5 Hz, H-26); 5.236 (d, J = 8.9 Hz, H-20); 4.606 (d, J = 8.9 Hz, H-20); 4.606 (db, J = 5.3 Hz, H-2); 4.424 (db, J = 13.4 Hz, H-6e); 3.69 (dxd, J = 9.6 and 1.1 Hz, H-14); 0.996/0.928/0.882 (d/d/d, J = 6.4/6.4/7.1 Hz, 11-, 17- and 25-$CH_3$);

Example 16: mixture of conformers: 5.41 (d, J = 9 Hz, H-29); 4.82 (d, J = 10 Hz, H-20); 4.44 (H-2); 4.42 (H-6 equ.); 4.04 (H-24); 3.82 (dxd, J = 1.5 and 9 Hz, H-14); 3.46 (d, J = 6 Hz, $-CH_2OSi$); 3.40 and 3.32 (s and s, 2x-$OCH_3$); 0.9 and 0.88 (s and s, 2x t-butyl); 0.04 (s, Si-$CH_3$);

Example 17: mixture of conformers: 5.43 (d, J = 9 Hz, H-29); 4.82 (d, J = 10 Hz, H-20); 4.44 (H-2); 4.41 (H-6 equ.); 4.22 (d, J = 7 Hz, $-CH_2$-OCOPhe); 4.05 (H-24); 3.82 (dxd, J = 1.5 and 9 Hz, H-14); 3.40 and 3.31 (s and s, 2x-$OCH_3$); 0.88 (s,t-butyl);

Example 18: mixture of conformers: 3.945 (d, J = 7.5 Hz, $-CH_2OAc$); 2.06 (s, $CH_3C{=}O$);

Example 19: mixture of conformers: 5.41 (d, J = 9 Hz, H-29); 4.82 (d, J = 10 Hz, H-20); 4.44 (H-2); 4.41 (H-6 equ.); 4.04 (H-24); 3.95 (d, J = 7 Hz, $-CH_2OC{=}O$); 3.82 (dxd, J = 1.5 and 9 Hz, H-14); 3.40 and 3.32 (s and s, 2x-$OCH_3$); 1.20 (s, t-butyl-C=O);

$^1H$-NMR:

Example 20: mixture of conformers: 2.36 [s, $N(CH_3)_2$]; 3.18 (s, $N-CH_2-CO$);

Example 21: mixture of conformers: 3.96 (d, J = 7 Hz, $-CH_2OCO$); 2.55 [sept, J = 7 Hz, $-CH(CH_3)_2$]; 1.18 (d, J = 7 Hz, $2x-CH_3$);

Example 22: mixture of conformers: 4.61 (db, J = 4 Hz, H-2); 4.44 (db, J = 13 Hz, H-6 equ.); 4.22 (d, J = 7 Hz, $-CH_2OCOPhe$); 3.92 (m, H-24); 3.68 (d, J = 9 Hz, H-14); 3.40 and 3.31 (2x s, $2x-OCH_3$);

Example 23: mixture of conformers: 2.06 (s, $CH_3C{=}O$); 3.95 (d, $-CH_2OAc$);

Example 24: mixture of conformers: 3.95 (d, J = 7 Hz, $-CH_2OC{=}O$); 1.21 (s, t-butyl-C=O);

Example 26: mixture of rotamers: 2.70 (m, $-CO-CH_2CH_2-CO-$);

Example 28: mixture of rotamers: 5.31 (d, J = 3.8 Hz, H-26); 4.59 (m, H-2); 4.42 (db, J = 13.5 Hz, H-6 equ.); approx. 4 (m, $CH_2-O-CO-$);

Example 30: mixture of conformers: 4.02 (d, $-CH_2OC{=}O$).

The compounds of the invention possess pharmacological activity. They are indicated for use as pharmaceuticals.

In particular they possess immunosuppressant and antiproliferative, and antiinflammatory activity.

Antiinflammatory activity may e.g. be determined in the following test methods:

1. Oxazolone-induced allergic contact dermatitis (mouse)

[the test method is as described in F.M. Dietrich and R. Hess, Int.Arch.Allergy 38 (1970) 246-259]:

The compounds elicit in this test an activity (inhibition of inflammatory swelling) between about 20 % and about 63 % upon a single topical application as a 0.01 % solution.

2. DNFB-induced allergic contact dermatitis (domestic pig)

(the test method is as described in e.g. EP 315978):

Two topical applications of a 0.4 % formulation of the compounds result in inhibition of the inflammatory reaction by from about 16 % to about 52 %.

3. Inhibition of phorbol ester (TPA) - induced irritant contact dermatitis (mouse)

(the test method is as described in e.g. EP 315978):

The compounds elicit in this test upon single application of a 3.6 % formulation an inhibition of the inflammatory reaction of from about 10 % to about 30 %.

Immunosuppressant and antiproliferative activity may e.g. be determined in the following test methods:

1. Proliferative response of lymphocytes to allogen stimulation in the mixed lymphocyte reaction (MLR) in vitro

[T. Meo, "The MLR in the Mouse", Immunological Methods, L. Lefkovits and B. Pernis, Eds., Academic Press, N.Y. (1979), 227-239]:

The compounds elicit in this test suppression of mixed lymphocytes ($IC_{50}$) at a dosage of from about < 0.0008 μg/ml to about 0.09 μg/ml.

2. Inhibition of the primary humoral immune response to sheep erythrocytes in vitro

[the test method is as described in R.I. Mishell and R.W. Dutton, Science 153 (1966) 1004-1006; R.I. Mishell and R.W. Dutton, J.Exp.Med. 126 (1967) 423-442]:

The compounds are active in this test with an $IC_{50}$ of from about 0.0024 μg/ml to about 0.32 μg/ml.

3. Inhibition of proliferation of human keratinocytes

(the test method is as described in e.g. EP 315978):

The compounds are active in this test at concentrations of from about 1 μg/ml to about 10 μg/ml, resulting in an inhibition of from about 30 % to about 90 %.

The compound of Examples 1 and 12 (32-hydroxymethyl-cyclopentyl-FR 520) is the preferred compound as an immunosuppressant agent. It has, for example, been determined that in the above DNFB allergic contact dermatitis (domestic pig) test this compound in the form of a 0.4 % preparation has better activity than a corresponding 1.2 % preparation of dexamethasone. It is, therefore, indicated that for this indication the compound of Examples 1 and 12 may be administered to larger mammals, for example humans, by similar modes of administration at similar or lower dosages than conventionally employed with dexamethasone.

The compounds of the invention in free form and where such forms exist in pharmaceutically acceptable salt form are therefore indicated as immunosuppressant and antiproliferative agents and as antiinflammatory agents for use in the prevention and treatment of conditions requiring immunosuppression and of inflammatory

conditions, such as

a) the prevention and treatment of

– resistance in situations of organ or tissue transplantation, e.g. of heart, kidney, liver, bone marrow and skin,

– graft-versus-host disease, such as following bone marrow grafts,

– autoimmune diseases such as rheumatoid arthritis, systemic Lupus erythematosus, Hashimoto's thyroidis, multiple sclerosis, Myasthenia gravis, diabetes type I and uveitis,

– skin manifestations of immunologically-mediated disorders;

b) the treatment of inflammatory and hyperproliferative skin diseases, such as psoriasis, atopical dermatitis, contact dermatitis and further eczematous dermatoses, seborrhoeic dermatitis, Lichen planus, Pemphigus, bullous Pemphigoid, Epidermolysis bullosa, urticaria, angioedemas, vasculitides, erythemas, cutaneous eosinophilias, Lupus erythematosus and acne; and

c) Alopecia areata.

The compounds may be administered systemically or topically. For the above indications the appropriate dosage will, of course, vary depending upon, for example, the host, the mode of administration and the nature and severity of the condition being treated. However, in general, satisfactory results are indicated to be obtained systemically at daily dosages of from about 0.15 mg/kg to about 1.5 mg/kg animal body weight. An indicated daily dosage in the larger mammal is in the range from about 0.01 mg to about 100 mg, conveniently administered, for example, in divided doses up to four times a day or in retard form. For topical use satisfactory results are obtained with local administration of a 1 to 3 % concentration of active substance several times daily, e.g. 2 to 5 times daily. Examples of indicated galenical forms are lotions, gels and creams.

The compounds of the invention may be administered by any conventional route, in particular enterally, e.g. orally, e.g. in the form of tablets or capsules, or topically, e.g. in the form of lotions, gels or creams.

Pharmaceutical compositions e.g. for topical application comprising a compound of the invention in free form or where such forms exist in pharmaceutically acceptable salt form in association with at least one pharmaceutical acceptable carrier or diluent may be manufactured in conventional manner by mixing with a pharmaceutically acceptable carrier or diluent. Unit dosage forms contain, for example, from about 0.0025 mg to about 50 mg of active substance.

Topical administration is e.g. to the skin. A further form of topical administration is to the eye, for the treatment of immune-mediated conditions of the eye, such as: auto-immune diseases, e.g. uveitis, keratoplasty and chronic keratitis; allergic conditions, e.g. vernal conjunctivitis; inflammatory conditions and corneal transplants, by the topical administration to the eye surface of a compound of the invention in a pharmaceutically acceptable ophthalmic vehicle.

The ophthalmic vehicle is such that the compound is maintained in contact with the ocular surface for a sufficient time period to allow the compound to penetrate the corneal and internal regions of the eye, e.g. the anterior chamber, posterior chamber, vitreous body, aqueous humor, vitreous humor, cornea, iris/ciliary, lens, choroid/retina and sclera.

The pharmaceutically acceptable ophthalmic vehicle may be e.g. an ointment, vegetable oil, or an encapsulating material.

Whilst the antiinflammatory and immunosuppressant and antiproliferative activity is the main activity of the compounds of the invention they also possesses some degree of activity in increasing sensitivity to, or in increasing the efficacy of, chemotherapeutic drug therapy. This activity may e.g. be determined according to the test methods described in EP 360760.

The compounds of the invention are therefore indicated for use in reversing chemotherapeutic drug resistance of varying types, e.g. acquired or innate, or in increasing sensitivity to administered drug therapy, e.g. as a means of reducing regular chemotherapeutic dosage levels, for example in the case of anti-neoplastic or cytostatic drug therapy, as a means of decreasing overall drug toxicity and, more especially, as a means of reversing or reducing resistance, including both inherent and acquired resistance, to chemotherapy.

The invention thus also concerns the use of a compound of the invention as a pharmaceutical, a such compound for use as a pharmaceutical, the use of a such compound for the preparation of a pharmaceutical compositon which comprises mixing with at least one pharmaceutically acceptable carrier or diluent, and a process for the preparation of a pharmaceutical composition which comprises mixing a compound of the invention together with at least one pharmaceutically acceptable carrier or diluent.

## Claims

1. A 12-(2'-cyclopentylvinyl)-11,28-dioxa-4-azatricyclo[22.3.1.$0^{4,9}$]octacos-18-ene or -14,18-diene

or an 11-(2′-cyclopentylvinyl)-10,27-dioxa-4-azatricyclo[21.3.1.0$^{4,8}$]heptacos-17-ene or -13,17-diene derivative,
with the proviso that the cyclopentyl moiety thereof is other than 3-formylcyclopentyl.

2. A compound according to claim 1 which is
a 17-alkyl (or 17-alkenyl)-1-hydroxy-14-oxy-12-(2′-cyclopentyl-1′-methyl-trans-vinyl)-23,25-dimethoxy-13,19,21,27-tetramethyl-11,28-dioxa-4-azatricyclo[22.3.1.0$^{4,9}$]octacos-18- trans-ene-2,3,10,16-tetraone or 17-alkyl (or 17-alkenyl)-1-hydroxy-12-(2′-cyclopentyl-1′-methyl-trans-vinyl)-23,25-dimethoxy-13,19,21,27-tetramethyl-11,28-dioxa-4-azatricyclo[22.3.1.0$^{4,9}$]octacos-14,18- trans-diene (or -18-trans-ene)-2,3,10,16-tetraone derivative,
with the proviso that the cyclopentyl moiety thereof is other than 3-formylcyclopentyl.

3. A compound according to claim 1 which is
a 17α-alkyl (or 17α-alkenyl)-1β-hydroxy-14α-oxy-12-[2′-(cyclopent-1″(R)-yl)-1′-methyl-trans-vinyl]-23α,25β-dimethoxy-13α,19,21α,27β-tetramethyl-11,28-dioxa-4-azatricyclo [22.3.1.0$^{4,9}$]octacos-18-trans-ene-9αH,24βH-2,3,10,16-tetraone
or 17α-alkyl (or 17α-alkenyl)-1β-hydroxy-12-[2′-(cyclopent-1″(R)-yl)-1′-methyl-trans-vinyl]-23α,25β-dimethoxy-13α,19,21α,27β-tetramethyl-11,28-dioxa-4-azatricyclo [22.3.1.0$^{4,9}$]octacos-14,18-trans-diene (or -18-trans-ene)-9αH,24βH-2,3,10,16-tetraone derivative,
with the proviso that the cyclopentyl moiety thereof is other than 3-formylcyclopentyl.

4. A compound according to claim 1 of formula I

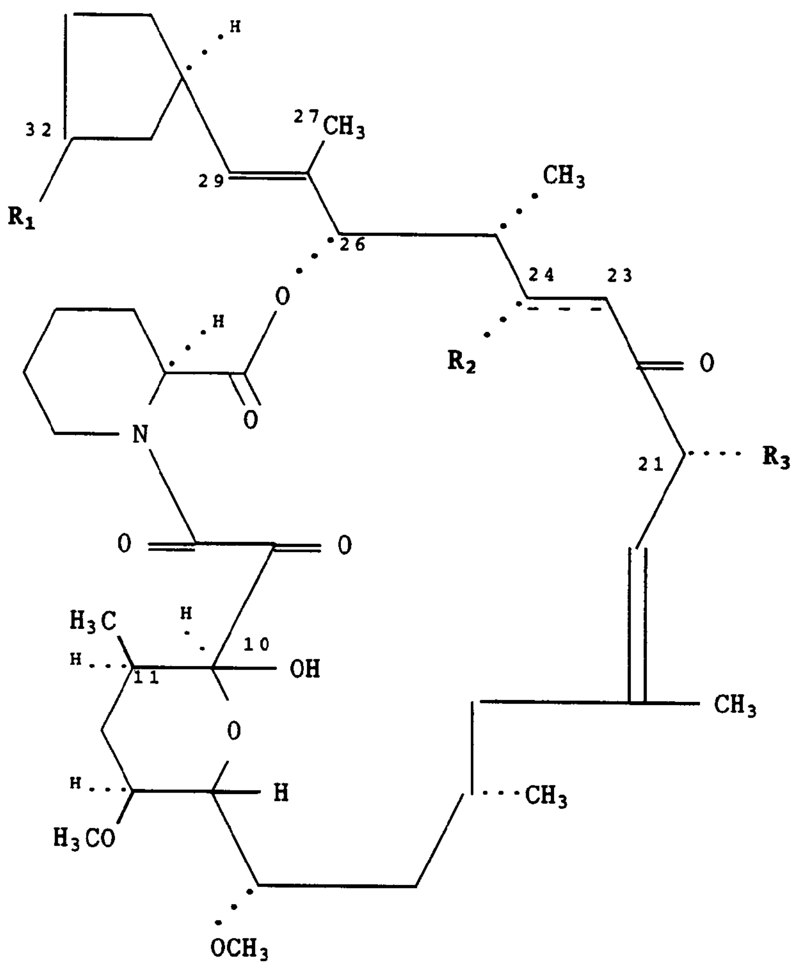

I

wherein
$R_1$ is methoxymethyl; optionally protected hydroxymethyl; acyloxymethyl wherein the acyl moiety optionally contains either a dimethylamino group which may optionally be quaternized, or a carboxy group

which may optionally be esterified, or one or more amino and/or hydroxy groups which may optionally be protected; or aminooxalyloxymethyl;
either $R_2$ is methoxy, formyloxy or optionally protected hydroxy and there is a single bond between positions 23 and 24
or $R_2$ is absent and there is a double bond between positions 23 and 24; and
$R_3$ is methyl, ethyl, n-propyl or allyl;
in free form and, where such forms exist, in salt form.

**5.** A compound according to claim 1 which is a compound $Ip_1$, i.e. a compound of formula I as defined in claim 4 wherein
$R_1$ is hydroxymethyl;
either $R_2$ is optionally protected hydroxy and there is a single bond between positions 23 and 24
or $R_2$ is absent and there is a double bond between positions 23 and 24; and
$R_3$ is as defined in claim 4 under formula I.

**6.** A compound according to claim 1 which is a compound $Ip_2$, i.e. a compound of formula I as defined in claim 4 wherein
$R_1$ is optionally protected hydroxymethyl;
$R_2$ is as defined in this claim for compounds $Ip_1$; and
$R_3$ is as defined in claim 4 under formula I;
in free form and, where such forms exist, in salt form.

**7.** A compound according to claim 1 which is a compound $Ip_3$, i.e. a compound of formula I as defined in claim 4 wherein
$R_1$ is the acyloxymethyl group formyloxymethyl;
either $R_2$ is formyloxy or optionally protected hydroxy and there is a single bond between positions 23 and 24
or $R_2$ is absent and there is a double bond between positions 23 and 24; and
$R_3$ is as defined in claim 4 under formula I,
in free form and, where such forms exist, in salt form.

**8.** A compound according to claim 1 which is a compound $Ip_4$, i.e. a compound of formula I as defined in claim 4 wherein
$R_1$ is acyloxymethyl wherein the acyl moiety optionally contains either a dimethylamino group which may optionally be quaternized, or one or more amino and/or hydroxy groups which may optionally be protected;
$R_2$ is as defined in this claim for compounds $Ip_1$; and
$R_3$ is as defined in claim 4 under formula I;
in free form and, where such forms exist, in salt form.

**9.** A compound according to claim 1 which is a compound $Ip_5$, i.e. a compound of formula I as defined in claim 4 wherein
$R_1$ is aminooxalyloxymethyl;
$R_2$ is as defined in this claim for compounds $Ip_1$; and
$R_3$ is as defined in claim 4 under formula I;
in free form and, where such forms exist, in salt form.

**10.** A compound according to claim 1 which is a compound $Ip_6$, i.e. a compound of formula I as defined in claim 1 wherein
either $R_1$ is methoxymethyl and
$R_2$ is methoxy or optionally protected hydroxy and there is a single bond between positions 23 and 24 or is absent and there is a double bond between positions 23 and 24;
or $R_1$ is optionally protected hydroxymethyl and
$R_2$ is methoxy; and
$R_3$ is as defined in claim 4 under formula I;
in free form and, where such forms exist, in salt form.

**11.** A compound according to claim 1 of formula Is

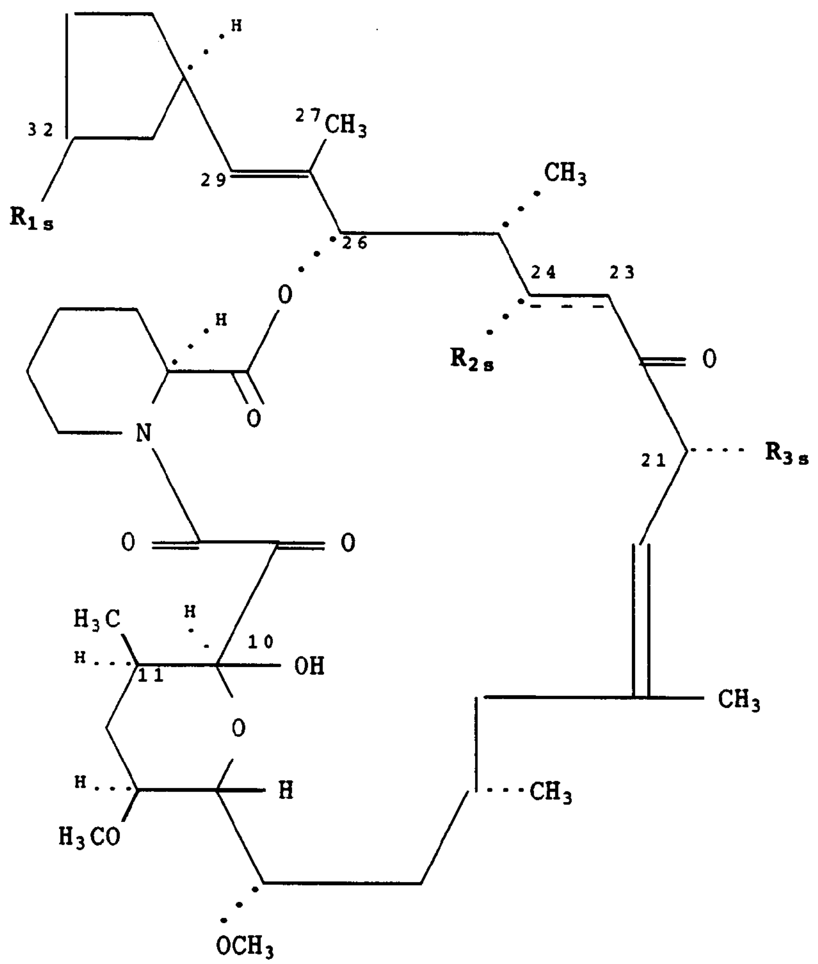

Is

wherein

$R_{1s}$ is methoxymethyl; hydroxymethyl optionally protected by tert-butyldimethylsilyl; benzoyloxymethyl; acyloxymethyl of 1 to 5 carbon atoms in the acyl moiety thereof, which optionally contains either a dimethylamino group which may optionally be quaternized or a carboxy group which may optionally be esterified by alkyl of 1 to 4 carbon atoms or an amino group which may optionally be protected by tert-butoxycarbonyl; or aminooxalyloxymethyl;

either $R_{2s}$ is methoxy, formyloxy or hydroxy optionally protected by tert-butyldimethylsilyl and there is a single bond between positions 23 and 24

or $R_{2s}$ is absent and there is a double bond between positions 23 and 24; and

$R_{3s}$ is ethyl or allyl,

in free form and, where such forms exist, in salt form.

**12.** The compound according to claim 1 which is 32-hydroxymethyl-cyclopentyl-FR520.

**13.** The compound according to claim 1 which is
either 24-O-tert-butyldimethylsilyl-32-hydroxymethyl-cyclopentyl-FR520,
or 32-tert-butyldimethylsilyloxymethyl-cyclopentyl-FR520,
or 24-O-formyl-32-formyloxymethyl-cyclopentyl-FR520,
or 32-aminooxalyloxymethyl-24-dehydroxy-$\Delta^{23}$-cyclopentyl-FR520,
or 32-aminooxalyloxymethyl-cyclopentyl-FR520,
or 24-methoxy-32-methoxymethyl-cyclopentyl-FR520,
or 24-O-tert-butyldimethylsilyl-32-isobutanoyloxymethyl-cyclopentyl-FR520,
or 24-O-tert-butyldimethylsilyl-32-[(N,N-dimethylamino)methyl]-carbonyloxymethyl-cyclopentyl-FR520,
or 32-[N-tert-butoxycarbonylamino)methyl]carbonyloxymethyl-24-O-tert-butyldimethylsilyl-cyclopentyl-FR520,
or 32-(N,N,N-trimethylammoniomethyl)carbonyloxymethyl-cyclopentyl-FR520 iodide,
or 32-isobutanoyloxymethyl-cyclopentyl-FR520,

in free form and, where such forms exist, in salt form.

**14.** The compound according to claim 1 of formula I wherein $R_1$ is hydroxymethyl and either $R_2$ is absent and there is a double bond between positions 23 and 24, and $R_3$ is ethyl,
or $R_2$ is hydroxy and there is a single bond between positions 23 and 24, and $R_3$ is allyl,
in free form and, where such forms exist, in salt form.

**15.** The compound according to claim 1 of formula I wherein there is a single bond between positions 23 and 24, $R_3$ is ethyl and either $R_1$ is $-CH_2OtBDMS$ and $R_2$ is OtBDMS,
or $R_1$ is benzoyloxymethyl and $R_2$ is OtBDMS,
or $R_1$ is acetoxymethyl and $R_2$ is OtBDMS,
or $R_1$ is pivaloyloxymethyl and $R_2$ is OtBDMS,
or $R_1$ is $(H_3C)_2NCH_2COOCH_2$- and $R_2$ is OH,
or $R_1$ is isobutanoyloxymethyl and $R_2$ is OH,
or $R_1$ is benzoyloxymethyl and $R_2$ is OH,
or $R_1$ is acetoxymethyl and $R_2$ is OH,
or $R_1$ is pivaloyloxymethyl and $R_2$ is OH,
or $R_1$ is BOC-$NHCH_2COOCH_2$- and $R_2$ is OH,
or $R_1$ is $HOOC(CH_2)_2COOCH_2$- and $R_2$ is OtBDMS,
or $R_1$ is $H_3COOC(CH_2)_2COOCH_2$- and $R_2$ is OtBDMS,
or $R_1$ is $HOOC(CH_2)_2COOCH_2$- and $R_2$ is OH,
or $R_1$ is $H_3COOC(CH_2)_2COOCH_2$- and $R_2$ is OH,
or $R_1$ is $H_2NCH_2COOCH_2$- and $R_2$ is OH,
in free form and, where such forms exist, in salt form.

**16.** A process for the preparation of a compound according to claim 1 which comprises subjecting a suitable 12-(2'-cyclohexylvinyl)-11,28-dioxa-4-azatricyclo[22.3.1.$0^{4,9}$]octacos-18-ene or -14,18-diene or a suitable 11-(2'-cyclohexylvinyl)-10,27-dioxa-4-azatricyclo[22.3.1.$0^{4,8}$]heptacos-17-ene or -13,17-diene derivative to ring contraction and appropriately derivatizing the resultant cyclopentyl compound.

**17.** A process according to claim 16 for the preparation of a compound of formula I as defined in claim 4 comprising

a) for the preparation of a compound of formula I wherein
$R_1$ is hydroxymethyl,
either $R_2$ is optionally protected hydroxy and there is a single bond between positions 23 and 24
or $R_2$ is absent and there is a double bond between positions 23 and 24, and
$R_3$ is as defined in claim 4 under formula I
(i.e. a compound Ia),
appropriately reducing a corresponding compound of formula II

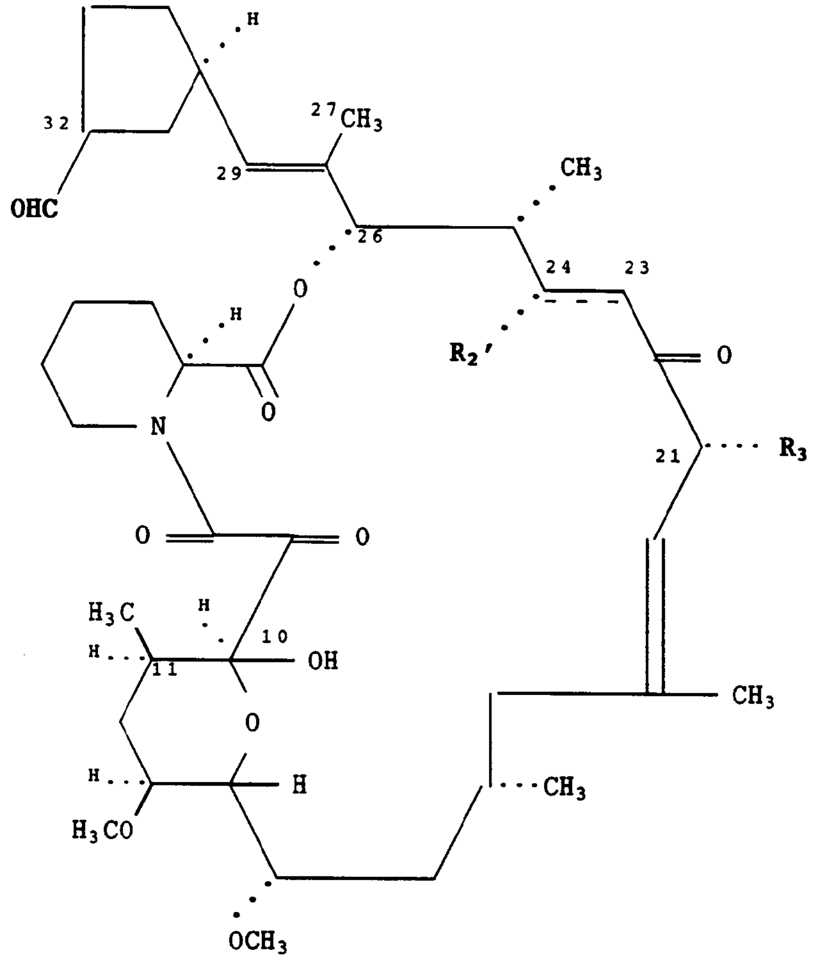

II

wherein
$R_2'$ either is optionally protected hydroxy and there is a single bond between positions 23 and 24, or is absent and there is a double bond between positions 23 and 24, and
$R_3$ is as defined in claim 4 under formula I;
b) for the preparation of a compound of formula I wherein
$R_1$ is protected hydroxymethyl,
either $R_2$ is optionally protected hydroxy and there is a single bond between positions 23 and 24
or $R_2$ is absent and there is a double bond between positions 23 and 24, and
$R_3$ is as defined in claim 4 under formula I, appropriately introducing a protecting group into a compound Ia;
c) for the preparation of a compound of formula I wherein
$R_1$ is optionally protected hydroxymethyl or formyloxymethyl,
either $R_2$ is formyloxy or optionally protected hydroxy and there is a single bond between positions 23 and 24
or $R_2$ is absent and there is a double bond between positions 23 and 24, and
$R_3$ is as defined in claim 4 under formula I,
with the proviso that at least one of $R_1$ and $R_2$ is formyloxymethyl or, respectively, formyloxy, appropriately formylating a corresponding compound of formula I wherein
$R_1$ is optionally protected hydroxymethyl or formyloxymethyl,
either $R_2$ is formyloxy or optionally protected hydroxy and there is a single between positions 23 and 24
or $R_2$ is absent and there is a double bond between positions 23 and 24, and
$R_3$ is as defined in claim 4 under formula I,
with the proviso that at least one of $R_1$ and $R_2$ is other than formyloxymethyl or, respectively, formyloxy;
d) for the preparation of a compound of formula I wherein
$R_1$ is aminooxalyloxymethyl,

either $R_2$ is optionally protected hydroxy and there is a single bond between positions 23 and 24
or $R_2$ is absent and there is a double bond between positions 23 and 24, and
$R_3$ is as defined in claim 4 under formula I,
treating with an appropriate oxalyl derivative and thereafter with ammonia a corresponding compound Ia;
e) for the preparation of a compound of formula I wherein
$R_1$ is methoxymethyl or optionally protected hydroxymethyl,
either $R_2$ is methoxy or optionally protected hydroxy and there is a single bond between positions 23 and 24
or $R_2$ is absent and there is a double bond between positions 23 and 24, and
$R_3$ is as defined in claim 4 under formula I,
with the proviso that at least one of $R_1$ and $R_2$ is methoxymethyl or, respectively, methoxy,
appropriately methylating a corresponding compound of formula I wherein
$R_1$ is optionally protected hydroxymethyl,
either $R_2$ is optionally protected hydroxy and there is a single bond between positions 23 and 24
or $R_2$ is absent and there is a double bond between positions 23 and 24, and
$R_3$ is as defined in claim 4 under formula I;
f) for the preparation of a compound of formula I wherein
$R_1$ is acyloxymethyl wherein the acyl moiety optionally contains either a dimethylamino group which may optionally be quaternized or a carboxy group which may optionally be esterified or one or more optionally protected amino and/or hydroxy groups,
either $R_2$ is optionally protected hydroxy and there is a single bond between positions 23 and 24
or $R_2$ is absent and there is a double bond between positions 23 and 24, and
$R_3$ is as defined in claim 4 under formula I
(i.e. a compound Ih),
appropriately acylating a corresponding compound Ia,
and if desired quaternizing a resultant compound Ih wherein $R_1$ contains a dimethylamino group;
and when a resultant compound of formula I has a protected hydroxy or hydroxymethyl and/or a protected amino group, optionally removing the protecting group(s) to give a corresponding compound of formula I having one or more unprotected hydroxy or hydroxymethyl and/or unprotected amino group(s);
and recovering the resultant compound of formula I in free form or, where such forms exist, in salt form.

**18.** A pharmaceutical composition comprising a compound according to claim 1 in association with at least one pharmaceutically acceptable carrier or diluent.

**19.** A compound according to claim 1 for use as a pharmaceutical.

**20.** Use of a compound according to claim 1 for the preparation of a pharmaceutical composition which comprises mixing with at least one pharmaceutically acceptable carrier or diluent.

**21.** A process for the preparation of a pharmaceutical composition which comprises mixing a compound according to claim 1 together with at least one pharmaceutically acceptable carrier or diluent.

**22.** Use as an intermediate of the cyclopentyl compound resulting from subjecting a suitable 12-(2′-cyclohexylvinyl)-11,28-dioxa-4-azatricyclo[22.3.1.$0^{4,9}$]octacos-18-ene or -14,18-diene or a suitable 11-(2′-cyclohexylvinyl)-10,27-dioxa-4-azatricyclo[22.3.1.$0^{4,8}$]heptacos-17-ene or -13,17-diene derivative to ring contraction.

**23.** Use as an intermediate according to claim 22 of a compound of formula II as defined in claim 17.

## Claims for the following Contracting States: ES and GR

**1.** A process for the preparation of
a 12-(2′-cyclopentylvinyl)-11,28-dioxa-4-azatricyclo[22.3.1.$0^{4,9}$]octacos-18-ene or -14,18-diene
or an 11-(2′-cyclopentylvinyl)-10,27-dioxa-4-azatricyclo[21.3.1.$0^{4,8}$]heptacos-17-ene or -13,17-diene derivative,
with the proviso that the cyclopentyl moiety thereof is other than 3-formylcyclopentyl,
which comprises subjecting a suitable 12-(2′-cyclohexylvinyl)-11,28-dioxa-4-azatricyclo[22.3.1.$0^{4,9}$]octacos-18-ene or -14,18-diene or a suitable 11-(2′-cyclohexylvinyl)-10,27-dioxa-4-azatricyclo [22.3.1.0$^{4,8}$] heptacos-17-ene or -13,17-diene derivative to ring contraction and appropriately derivatizing the resultant cyclopentyl compound.

**2.** A process according to claim 1 for the preparation of a 17-alkyl (or 17-alkenyl)-1-hydroxy-14-oxy-12-(2′-cyclopentyl-1′-methyl-trans-vinyl)-23,25-dimethoxy-13,19,21,27-tetramethyl-11,28-dioxa-4-azatricyclo[22.3.1.0$^{4,9}$]octacos-18- trans-ene-2,3,10,16-tetraone or 17-alkyl (or 17-alkenyl)-1-hydroxy-12-(2′-cyclopentyl-1′-methyl-trans-vinyl)-23,25-dimethoxy-13,19,21, 27-tetramethyl-11,28-dioxa-4-azatricyclo[22.3.1.0$^{4,9}$]octacos-14,18- trans-diene (or -18-trans-ene)-2,3, 10,16-tetraone derivative,
with the proviso that the cyclopentyl moiety thereof is other than 3-formylcyclopentyl.

**3.** A process according to claim 1 for the preparation of a 17α-alkyl (or 17α-alkenyl)-1β-hydroxy-14α-oxy-12-[2′-(cyclopent-1″(R)-yl)-1′-methyl-trans-vinyl]-23α, 25β-dimethoxy-13α,19,21α,27β-tetramethyl-11,28-dioxa-4-azatricyclo [22.3.1.0$^{4,9}$]octacos-18-trans-ene-9αH,24βH-2,3,10,16-tetraone
or 17α-alkyl (or 17α-alkenyl)-1β-hydroxy-12-[2′-(cyclopent-1″(R)-yl)-1′-methyl-trans-vinyl]-23α,25β-dimethoxy-13α,19,21α,27β-tetramethyl-11,28-dioxa-4-azatricyclo [22.3.1.0$^{4,9}$]octacos-14,18-trans-diene (or -18-trans-ene)-9αH,24βH-2,3,10,16-tetraone derivative,
with the proviso that the cyclopentyl moiety thereof is other than 3-formylcyclopentyl.

**4.** A process according to claim 1 for the preparation of a compound of formula I

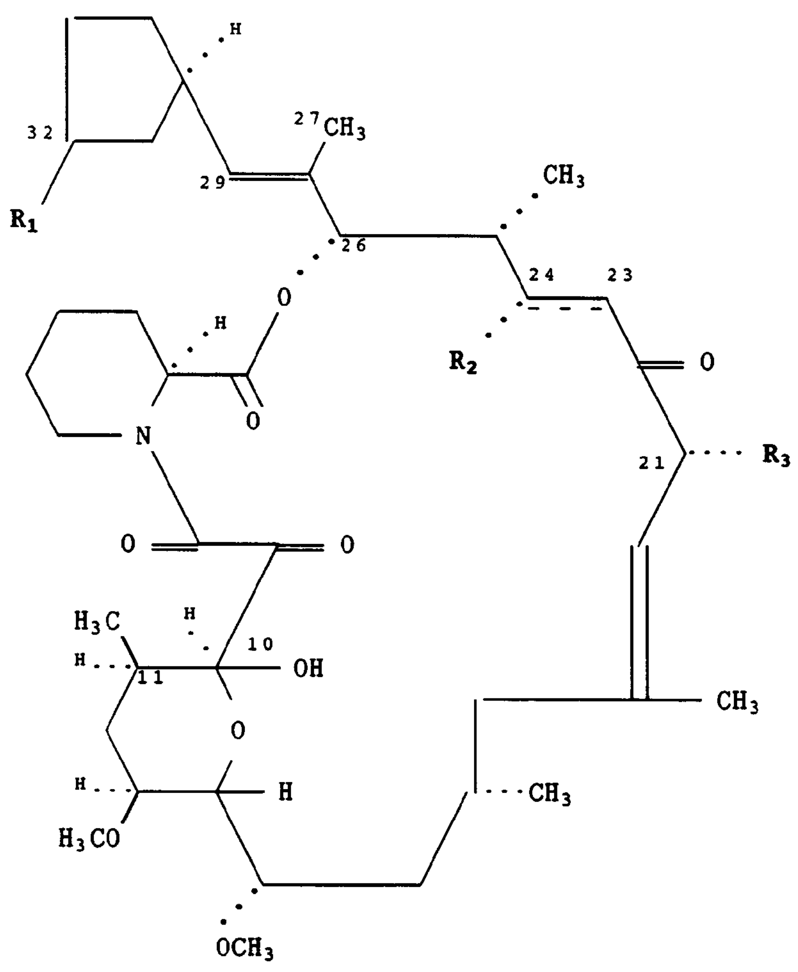

I

wherein
$R_1$ is methoxymethyl; optionally protected hydroxymethyl; acyloxymethyl wherein the acyl moiety optionally contains either a dimethylamino group which may optionally be quaternized, or a carboxy group

which may optionally be esterified, or one or more amino and/or hydroxy groups which may optionally be protected; or aminooxalyloxymethyl;
either $R_2$ is methoxy, formyloxy or optionally protected hydroxy and there is a single bond between positions 23 and 24
or $R_2$ is absent and there is a double bond between positions 23 and 24; and
$R_3$ is methyl, ethyl, n-propyl or allyl;
in free form and, where such forms exist, in salt form, which comprises

a) for the preparation of a compound of formula I wherein
$R_1$ is hydroxymethyl,
either $R_2$ is optionally protected hydroxy and there is a single bond between positions 23 and 24
or $R_2$ is absent and there is a double bond between positions 23 and 24, and
$R_3$ is as defined in this claim under formula I
(i.e. a compound Ia),
appropriately reducing a corresponding compound of formula II

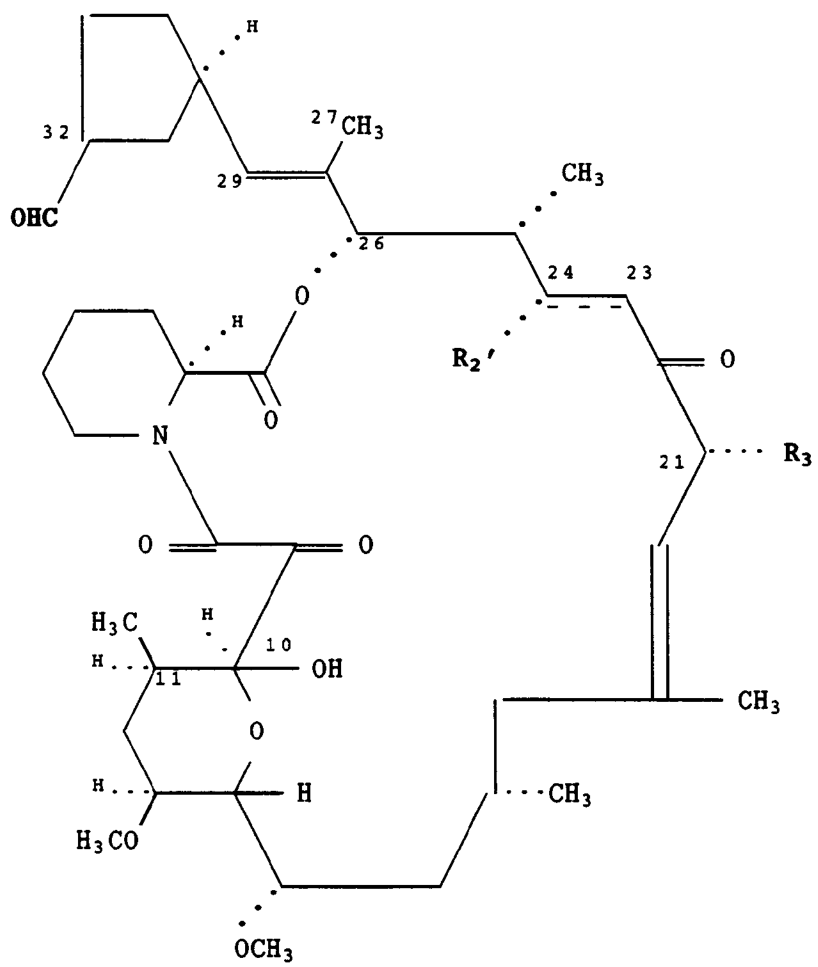

II

wherein
$R_2'$ either is optionally protected hydroxy and there is a single bond between positions 23 and 24, or is absent and there is a double bond between positions 23 and 24, and
$R_3$ is as defined in this claim under formula I;

b) for the preparation of a compound of formula I wherein
$R_1$ is protected hydroxymethyl,
either $R_2$ is optionally protected hydroxy and there is a single bond between positions 23 and 24
or $R_2$ is absent and there is a double bond between positions 23 and 24, and
$R_3$ is as defined in this claim under formula I,
appropriately introducing a protecting group into a compound Ia;

c) for the preparation of a compound of formula I wherein

$R_1$ is optionally protected hydroxymethyl or formyloxymethyl,
either $R_2$ is formyloxy or optionally protected hydroxy and there is a single bond between positions 23 and 24
or $R_2$ is absent and there is a double bond between positions 23 and 24, and
$R_3$ is as defined in this claim under formula I,
with the proviso that at least one of $R_1$ and $R_2$ is formyloxymethyl or, respectively, formyloxy,
appropriately formylating a corresponding compound of formula I wherein
$R_1$ is optionally protected hydroxymethyl or formyloxymethyl,
either $R_2$ is formyloxy or optionally protected hydroxy and there is a single between positions 23 and 24
or $R_2$ is absent and there is a double bond between positions 23 and 24, and
$R_3$ is as defined in this claim under formula I,
with the proviso that at least one of $R_1$ and $R_2$ is other than formyloxymethyl or, respectively, formyloxy;
d) for the preparation of a compound of formula I wherein
$R_1$ is aminooxalyloxymethyl,
either $R_2$ is optionally protected hydroxy and there is a single bond between positions 23 and 24
or $R_2$ is absent and there is a double bond between positions 23 and 24, and
$R_3$ is as defined in this claim under formula I,
treating with an appropriate oxalyl derivative and thereafter with ammonia a corresponding compound Ia;
e) for the preparation of a compound of formula I wherein
$R_1$ is methoxymethyl or optionally protected hydroxymethyl,
either $R_2$ is methoxy or optionally protected hydroxy and there is a single bond between positions 23 and 24
or $R_2$ is absent and there is a double bond between positions 23 and 24, and
$R_3$ is as defined in this claim under formula I,
with the proviso that at least one of $R_1$ and $R_2$ is methoxymethyl or, respectively, methoxy,
appropriately methylating a corresponding compound of formula I wherein
$R_1$ is optionally protected hydroxymethyl,
either $R_2$ is optionally protected hydroxy and there is a single bond between positions 23 and 24
or $R_2$ is absent and there is a double bond between positions 23 and 24, and
$R_3$ is as defined in this claim under formula I;
f) for the preparation of a compound of formula I wherein
$R_1$ is acyloxymethyl wherein the acyl moiety optionally contains either a dimethylamino group which may optionally be quaternized or a carboxy group which may optionally be esterified or one or more optionally protected amino and/or hydroxy groups,
either $R_2$ is optionally protected hydroxy and there is a single bond between positions 23 and 24
or $R_2$ is absent and there is a double bond between positions 23 and 24, and
$R_3$ is as defined in this claim under formula I
(i.e. a compound Ih),
appropriately acylating a corresponding compound Ia,
and if desired quaternizing a resultant compound Ih wherein $R_1$ contains a dimethylamino group;
and when a resultant compound of formula I has a protected hydroxy or hydroxymethyl and/or a protected amino group, optionally removing the protecting group(s) to give a corresponding compound of formula I having one or more unprotected hydroxy or hydroxymethyl and/or unprotected amino group(s);
and recovering the resultant compound of formula I in free form or, where such forms exist, in salt form.

**5.** A process according to claim 4 for the preparation of the compound of formula I which is 32-hydroxymethylcyclopentyl-FR520 in free form and, where such forms exist, in salt form.

**6.** A process for the preparation of a pharmaceutical composition comprising mixing a compound according to claim 1 with at least one pharmaceutically acceptable carrier or diluent.

**7.** Use as an intermediate of the cyclopentyl compound resulting from subjecting a suitable 12-(2′-cyclohexylvinyl)-11,28-dioxa-4-azatricyclo[22.3.1.$0^{4,9}$]octacos-18-ene or -14,18-diene or a suitable 11-(2′-cyclohexylvinyl)-10,27-dioxa-4-azatricyclo[22.3.1.$0^{4,8}$]heptacos-17-ene or -13,17-diene derivative to ring contraction.

8. Use as an intermediate according to claim 7 of a compound of formula II as defined in claim 4.

European Patent Office

# EUROPEAN SEARCH REPORT

Application Number

EP 91 81 0507

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X<br>P | EP-A-0 427 680 (SANDOZ LTD)<br>* Pages 32-39 * | 1-4,16, 18-23 | C 07 H 19/01<br>A 61 K 31/70 |
| A,D | EP-A-0 184 162 (FUJISAWA PHARMACEUTICAL CO., LTD)<br>* Pages 100-110 * | 1-4,18-21 | |
| A | EP-A-0 323 042 (FISONS PLC)<br>* Pages 18-21 * | 1-4,18-21 | |

| TECHNICAL FIELDS SEARCHED (Int. Cl.5) |
|---|
| C 07 H 19/00<br>A 61 K 31/00 |

The present search report has been drawn up for all claims

| Place of search | Date of completion of the search | Examiner |
|---|---|---|
| THE HAGUE | 25-09-1991 | DAY G.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)